# EUROPEAN PATENT SPECIFICATION

(11) **EP 1 978 967 B1**
(45) Date of publication and mention of the grant of the patent: **22.09.2010**
(21) Application number: 06829350.5
(22) Date of filing: 06.12.2006
(51) Int. Cl.: A61K 31/517, C07D 471/04, C07D 487/04, C07D 498/04, A61P 25/04

(54) **QUINAZOLINONE DERIVATIVES AS VANILLOID ANTAGONISTS**
CHINAZOLINON-DERIVATE ALS VANILLOID-ANTAGONISTEN
DÉRIVÉS DE QUINAZOLINONE COMME ANTAGONISTES DE LA VANILLOÏDE

(30) Priority: 08.12.2005 GB 0525069
(43) Date of publication of application: 15.10.2008
(73) Proprietor: Novartis AG, 4002 Basel (CH)
(72) Inventor: DZIADULEWICZ, Edward, Karol, Horsham, West Sussex RH12 5AB (GB); BRAIN, Christopher, Thomas, Horsham, West Sussex RH12 5AB (GB); RITCHIE, Timothy, John, Horsham, West Sussex RH12 5AB (GB); CULSHAW, Andrew, James, Horsham, West Sussex RH12 5AB (GB)
(74) Representative: Vögeli-Lange, Regina
(86) International application number: PCT/EP2006/011721
(87) International publication number: WO 2007/065663

(56) References cited:
- EP-A1- 1 199 307
- WO-A-2005/049613
- DATABASE CA [Online] CHEMICAL ABSTRACTS SERVICE, COLUMBUS, OHIO, US; KUMARI, T. ARUNA ET AL: "A facile synthesis of 2,3-disubstituted pyrido[2,3-h]quinazolin-4(3H)-ones" XP002424032 retrieved from STN Database accession no. 2002:132282 & SYNTHETIC COMMUNICATIONS , 32(2), 235-240 CODEN: SYNCAV; ISSN: 0039-7911, 2002,
- DATABASE CA [Online] CHEMICAL ABSTRACTS SERVICE, COLUMBUS, OHIO, US; REDDY, M. SATYANARAYANA ET AL: "A facile synthesis of 3-aryl-2-styryl- and 3-aryl-2-methylpyrido[2,3- h]quinazolin-4(3H)-ones" XP002424033 retrieved from STN Database accession no. 1984:455033 & INDIAN JOURNAL OF CHEMISTRY, SECTION B: ORGANIC CHEMISTRY INCLUDING MEDICINAL CHEMISTRY , 22B(11), 1100-2 CODEN: IJSBDB; ISSN: 0376-4699, 1983,

## Description

The present invention relates to quinazolinone derivatives as vanilloid antagonists, to processes for preparing them, to their use as pharmaceuticals and to pharmaceutical compositions containing them.

In a first aspect, the present invention provides a quinazolinone compound of the formula 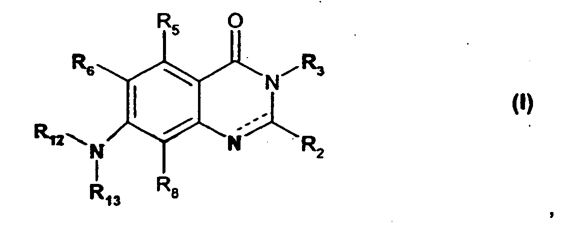 **wherein**
---- is a single bond or a double bond;
R₂ is selected from
(a) C₁-C₈alkyl, C₃-C₆cycloalkyl, (C₁-C₆alkyl)amino or di-(C₁-C₆alkyl)amino;
   or
(b) NH₂, hydroxyC₁-C₆alkylamino-, aminoC₁-C₆alkylamino, C₂-C₆alkenyl, di(tifluoromethyl)C₁-C₆alkyl, R₉-O-(C₁ -C₆alkyl)- in which the alkyl chain is optionally substituted by trifluoromethyl, (NC)-C₁ -C₆alkyl-, (R₁₀R₁₁N-)C₁ - C₆alkyl-, (C₁ -C₆alkyl)-SO₂-(C₁-C₆alkyl)-, wherein R₉, R₁₀ and R₁₁ are each independently H or C₁-C₆ alkyl; phenyl optionally substituted by one, two or three substituents each independently selected from the group consisting of halogen, C₁ -C₆alkyl, halogen-substituted C₁ -C₆alkyl, hydroxy C₁ -C₆alkyl, cyano or a group -(C=O)-R₂ₐ, where R₂ₐ is C₁ -C₆alkyl; or 5, 6, or 7-membered, saturated or unsaturated, heterocyclic ring, directly attached to the quinazolinone ring or attached through -C₁ -C₆ alkyl-, containing one, two, or three heteroatoms selected from N, O and S, and optionally substituted with one, two or three substitutents selected from C₁ -C₆alkyl, C₁ -C₆alkoxy, hydroxy, cyano, halo, R₁₀R₁₁N-, R₉-O-(C=O)-, -(C=O)-N-R₁₀R₁₁, =O and phenyl ;
R₃ is selected from
(a'):
   phenyl substituted by one, two or three substituents each independently selected from the group consisting of halogen, C₁-C₆alkyl, halogen-substituted C₁-C₆alkyl, hydroxyC₁-C₆alkyl, cyano or a group -C(=O)-R₃ₐ, where R₃ₐ is C₁-C₆alkyl; or
(b'):
   C₁ -C₆alkyl, (NC)-C₁ -C₆alkyl-, R₉-O-(C₁ -C₆alkyl)-, R₉-O-( C₁ -C₆alkyl)-O-(C₁ - C₆alkyl)-,R₁₀R₁₁N-(C₁ -C₆alkyl)-, R₁₀R₁₁N-(C=O)-(C₁ -C₆alkyl)-, or (C₁ - C₆alkyl)-SO₂-(C₁ -C₆alkyl)-, wherein R₉, R₁₀ and R₁₁ are each independently H or C₁-C₆ alkyl; or
unsubstituted phenyl, phenyl substituted with one or two substituents selected from -(C₁ -C₆alkoxy)-, R₁₀R₁₁N-, R₁₀R₁₁N-(C₁ -C₆alkyl)-, -SO₂-(C₁-C₆alkyl); R₉-O-(C=O)-, wherein R₉, R₁₀ and R₁₁ are as defined above, or with halo-substituted phenyl or a 5- or 6-membered saturated or unsaturated heterocyclic ring having one, two or three heteroatoms selected from N, O and S and optionally including a further substituent selected from halo, or phenyl substituted with three or four substituents selected from halo, hydroxyl, and C₁ -C₆alkyl; or
a cycloalkyl ring having 3, 4, 5 or 6 carbon atoms, directly attached to the quinazolinone ring or attached through -C₁ -C₆alkyl-, and which is optionally substituted with one or two substituents selected from C₁ -C₆alkyl, C₁ - C₆alkoxy, hydroxy, cyano, halo, R₁₀R₁₁N-, R₉-O-(C=O)-, -(C=O)-N-R₁₀R₁₁, and phenyl; or
benzyl, or phenyl(C₁ -C₆alkyl)-, phenoxy-( C₁ -C₆alkyl)- or phenyl(C=O)-(C₁ - C₆alkyl)-, optionally substituted with one, two, or three substituents selected from C₁ -C₆alkyl, C₁ -C₆alkoxy, hydroxy, cyano, halo, R₁₀R₁₁N-, R₉-O-(C=O)-, -(C=O)-N-R₁₀R₁₁, and phenyl; or
a 5, 6, or 7- membered, saturated or unsaturated, heterocyclic ring, directly attached to the quinazolinone ring or attached through -C₁ -C₆ alkyl-, containing one, two, or three heteroatoms selected from N, O and S, and optionally substituted with one, two or three substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, =O and phenyl; or
a 9- or 10- membered aromatic or heterocyclic fused ring, directly attached to the quinazolinone ring or attached through -C₁-C₆ alkyl-, containing zero, one, two or three heteroatoms selected from N, O and S, and optionally substituted with one, two, three or four substitutents selected from C₁-C₆alkyl, C₁-C₆alkoxy, hydroxy, cyano, halo, R₁₀R₁₁N-, R₉-O-(C=O)-, -(C=O)-N-R₁₀R₁₁, and phenyl; and
R₅ and R₆ are each independently hydrogen, halogen, C₁-C₆alkyl, C₂-C₆alkenyl, C₂-C₆alkynyl, hydroxy, hydroxy-substituted C₁-C₆alkyl, C₁-C₆alkoxy, C₃-C₆cycloalkyl, cyano, -C(=O)H, phenyl, (C₃-C₆cycloalkyl)C₁-C₆alkyl, (C₃-C₆cycloalkyl)C₁-C₆alkoxy, (C₁-C₆alkoxycarbonylamino)C₁-C₆alkoxy or (C₁-C₆alkylcarbonylamino)C₁-C₆alkoxy, (amino) C₁-C₆alkoxy, (dimethylamino)C₁-C₆alkoxy, or (C₁-C₆alkoxycarbonyl) C₁-C₆alkoxy;
R12 is hydrogen, formyl, C₁-C₆alkylcarbonyl or benzyl, the phenyl group of which is optionally substituted by 1, 2 or 3 substituents, selected from the group consisting of halogen, C₁-C₆alkyl, halo-C₁-C₆alkyl, hydroxy, hydroxy-C₁-C₆alkyl, C₁-C₆alkoxy, C₁-C₆alkoxy-C₁-C₆alkyl, halo-C₁-C₆alkoxy, C₁-C₆alkylthio, halo-C₁-C₆alkylthio, C₁-C₆alkylsulfinyl, halo-C₁-C₆alkylsulfinyl, C₁-C₆alkylsulfonyl, halo-C₁-C₆alkylsulfonyl, C₃-C₆cycloalkyl, C₃-C₆cycloalkyl-C₁-C₆alkyl, C₃-C₆cycloalkoxy, C₃-C₆cycloalkoxy-C₁-C₆alkyl, amino, C₁-C₆alkylamino, di-(C₁-C₆alkyl)amino, C₁-C₆alkoxycarbonylamino, cyano, formyl and C₁-C₆alkylcarbonyl, or is substituted at two adjacent carbon atoms by -O-CH₂-O- or -O-CF₂-O-; and
R13 and R8, taken together, represent, together with the three-membered moiety -N-C-C-, to which they are attached, a five-, six-, seven- or eight-membered, partially or fully unsaturated, optionally substituted, heterocyclic ring, which contains 1 ring nitrogen atom and optionally either 1 further ring nitrogen, oxygen or sulfur atom or 2 further ring nitrogen atoms, in which heterocyclic ring each ring oxygen or sulfur atom is bonded to 2 ring carbon atoms, the optional substituents of the said heterocyclic ring being selected from the group consisting of halogen, C₁-C₆alkyl, halo-C₁-C₆alkyl, hydroxy-C₁-C₆alkyl, C(O)-C₁-C₆alkyl and oxo,
in free form or in salt form,
provided that the compound of formula I is not
pyrido[2,3-h]quinazolin-4(3H)-one, 2,3-diphenyl;
pyrido[2,3-h]quinazolin-4(3H)-one, 3-(4-nitrophenyl)-2-phenyl;
pyrido[2,3-h]quinazolin-4(3H)-one, 3-(4-methylphenyl)-2-phenyl;
pyrido[2,3-h]quinazolin-4(3H)-one, 3-(2-methylphenyl)-2-phenyl;
pyrido[2,3-h]quinazolin-4(3H)-one, 2-(4-chlorophenyl)-3-phenyl;
pyrido[2,3-h]quinazolin-4(3H)-one, 2-(4-chlorophenyl)-3-(4-nitrophenyl);
pyrido[2,3-h]quinazolin-4(3H)-one, 2-(4-chlorophenyl)-3-(4-methylphenyl);
pyrido[2,3-h]quinazolin-4(3H)-one, 2-(4-chlorophenyl)-3-(2-methylphenyl);
pyrido[2,3-h]quinazolin-4(3H)-one, 2-(4-methylphenyl)-3-phenyl;
pyrido[2,3-h]quinazolin-4(3H)-one, 2-(4-methylphenyl)-3-(4-nitrophenyl);
pyrido[2,3-h]quinazolin-4(3H)-one, 2,3-bis(4-methylphenyl);
pyrido[2,3-h]quinazolin-4(3H)-one, 3-(2-methylphenyl)-2-(4-methylphenyl);
pyrido[2,3-h]quinazolin-4(3H)-one, 2-(4-methoxyphenyl)-3-phenyl;
pyrido[2,3-h]quinazolin-4(3H)-one, 2-(4-methoxyphenyl)-3-(4-nitrophenyl);
pyrido[2,3-h]quinazolin-4(3H)-one, 2-(4-methoxyphenyl)-3-(4-methylphenyl);
pyrido[2,3-h]quinazolin-4(3H)-one, 2-(4-methoxyphenyl)-3-(2-methylphenyl);
pyrido[2,3-h]quinazolin-4(3H)-one, 2-(4-methylphenyl);
pyrido[2,3-h]quinazolin-4(3H)-one, 2-(4-methylphenyl)-3-(phenylmethyl);
pyrido[2,3-h]quinazolin-4(3H)-one, 3-methyl-2-(4-methylphenyl);
pyrido[2,3-h]quinazolin-4(3H)-one, 3-phenyl-2-(2-phenylethenyl);
pyrido[2,3-h]quinazolin-4(3H)-one, 2-[2-(4-methoxyphenyl)ethenyl]-3-phenyl;
pyrido[2,3-h]quinazolin-4(3H)-one, 2-[2-(4-methylphenyl)ethenyl]-3-phenyl;
pyrido[2,3-h]quinazolin-4(3H)-one, 2-[2-(4-chlorophenyl)ethenyl]-3-phenyl;
pyrido[2,3-h]quinazolin-4(3H)-one, 2-[2-(4-nitrophenyl)ethenyl]-3-phenyl;
pyrido[2,3-h]quinazolin-4(3H)-one, 3-(4-methoxyphenyl)-2-(2-phenylethenyl);
pyrido[2,3-h]quinazolin-4(3H)-one, 3-(4-methylphenyl)-2-(2-phenylethenyl);
pyrido[2,3-h]quinazolin-4(3H)-one, 3-(3-nitrophenyl)-2-(2-phenylethenyl);
pyrido[2,3-h]quinazolin-4(3H)-one, 3-(4-methoxyphenyl)-2-[2-(4-methoxyphenyl)ethenyl];
pyrido[2,3-h]quinazolin-4(3H)-one, 3-(4-methoxyphenyl)-2-[2-(4-methylphenyl)ethenyl];
pyrido[2,3-h]quinazolin-4(3H)-one, 3-(4-chlorophenyl)-2-[2-(4-chlorophenyl)ethenyl];
pyrido[2,3-h]quinazolin-4(3H)-one, 2-methyl-3-phenyl;
pyrido[2,3-h]quinazolin-4(3H)-one, 3-(4-methoxyphenyl)-2-methyl;
pyrido[2,3-h]quinazolin-4(3H)-one, 2-methyl-3-(2-methylphenyl);
pyrido[2,3-h]quinazolin-4(3H)-one, 2-methyl-3-(4-methylphenyl);
pyrido[2,3-h]quinazolin-4(3H)-one, 3-(2-chlorophenyl)-2-methyl;
pyrido[2,3-h]quinazolin-4(3H)-one, 3-(4-chlorophenyl)-2-methyl;
pyrido[2,3-h]quinazolin-4(3H)-one, 2-methyl-3-(phenylmethyl)

If at least one asymmetrical carbon atom is present in a compound of the formula I, such a compound may exist in optically active form or in the form of mixtures of optical isomers, e. g. in the form of racemic mixtures. All optical isomers and their mixtures, including the racemic mixtures, are part of the present invention.

Compounds of formula I are useful as vanilloid antagonists, that is, they exhibit human vanilloid antagonist activity, and, more particularly, they demonstrate antagonism at the TRPVI receptor. As such they are indicated in the treatment of diseases and conditions in which vanilloid receptor activity plays a role or is indicate.

In the compounds of formula I, certain substituents may be preferred, independently, collectively, or in any combination or sub-combination.

For example, ---- is preferably a double bond.

In certain embodiments, in the compound of formula I, R₂ may preferably be C₁-C₈alkyl or cycloalkyl, more preferably C₁-C₆ alkyl, for example C₁-C₄ alkyl. One particularly preferred value for R₂ is isopropyl. In other embodiments, R₂ may preferably be NH₂ or C₂-C₆alkenyl, for example C₂-C₄alkenyl, such as isopropenyl. When R₂ is a heterocyclic ring as described above it is preferably 5- or 6- membered with one or two heteroatoms selected from N, O and S; a preferred subsituent for the heterocyclic ring is C₁-C₆alkyl, for example C₁-C₄alkyl such as methyl; where the heterocyclic ring is attached to the quinazolinone ring via C₁-C₆alkyl, C₁-C₄alkyl such as propyl, ethyl, and, most preferably methyl, is preferred. Examples of suitable heterocyclic rings include pyridine, furanyl, isoxazole, pyrrolidone, imidazole, thiophene, morpholine, pyrazine, pyrrole, piperidine and thiazole;

When R₃ is C₁ -C₆alkyl, (NC)-C₁ -C₆alkyl-, R₉-O-(C₁ -C₆alkyl)-, R₉-O-( C₁ -C₆alkyl)-O-(C₁-C₆alkyl)-,R₁₀R₁₁N-(C₁-C₆alkyl)-, R₁₀R₁₁N-(C=O)-(C₁-C₆alkyl)-, or (C₁ - C₆alkyl)-SO₂-(C₁-C₆alkyl)-, wherein R₉, R₁₀ and R₁₁ are each independently H or C₁-C₆ alkyl, it may preferably be one of the following:
C₁ -C₆alkyl, for example C₁ -C₄alkyl, such as isopropyl, propyl, methylbutyl; (NC)-C₁ -C₆alkyl-, for example (NC)-C₁ -C₄alkyl, such as acetonitrile;
R₉-O-(C₁ -C₆alkyl), for example R₉-O-(C₁ -C₄alkyl), such as hydroxyethyl, methoxyethyl;
R₁₀R₁₁N-(C₁ -C₆alkyl)-, for example R₁₀R₁₁N-(C₁ -C₄alkyl)-, such as dimethylaminoethyl, methylaminoethyl;
R₁₀R₁₁N-(C=O)-(C₁-C₆alkyl)-, such as R₁₀R₁₁N-(C=O)-(C₁-C₄alkyl), such as dimethylacetamide;
R₉-O-(C₁-C₆alkyl)-O-(C₁-C₆alkyl)-, such as R₉-O-(C₁-C₄alkyl)-O-(C₁-C₄alkyl)-, such as hydroxyethoxyethyl;
(C₁-C₆alkyl)-SO₂-(C₁-C₆alkyl)-, such as (C₁-C₄alkyl)-SO₂-(C₁-C₄alkyl)-, such as methylsulfonylethyl;
when R₃ is unsubstituted phenyl or phenyl substituted according to the above, it may preferably be one of the following: unsubstituted phenyl;
C₁-C₆ alkoxy phenyl, for example C₁-C₄ alkoxy phenyl, such as methoxyphenyl; or Phenyl substituted by halogen according to the above; such as phenyl substituted with halogen, for example chlorine, and with R₁₀R₁₁N-(C₁ -C₆alkyl)-, for example R₁₀R₁₁N-(C₁-C₄alkyl)-, such as dimethylaminomethyl, or phenyl substituted three or four times wherein the substituents are selected from halo, for example choro and fluoro, hydroxyl, methoxy, trifluoromethyl and methyl;
Phenyl substituted with a 5- or 6-membered saturated or unsaturated heterocyclic ring having one, two or three heteroatoms selected from N, O and S, for example oxazole, or
Phenyl substituted with halo-substituted phenyl, for example fluoro-biphenyl;
when R₃ is cycloalkyl as defined above it may preferably be one of the following: C₃-C₆ cycloalkyl directly attached to the quinzolinone ring, for example cyclopropyl, cyclobutyl, cyclopentyl or cyclohexyl;
C₃-C₆ cycloalkyl attached to the quinzolinone ring via C₁ -C₆alkyl, for example C₁ - C₄alkyl, such as propyl, isopropyl, ethyl or, particularly, methyl;

Substituted C₃-C₆ cycloalkyl having for example a single substitutent selected from - (C=O)OR₉, for example -(C=O)OC₁-C₆alkyl such as -(C=O)OC₁-C₄alkyl, for example -(C=O)OMe or, particularly, -(C=O)OEt;
when R₃ is benzyl, or phenyl(C₁ -C₆alkyl)-, phenoxy-( C₁ -C₆alkyl)- or phenyl(C=O)-(C₁ -C₆alkyl)-, each as defined above, it may preferably be one of the following: benzyl;
benzyl substituted by one or two substituents selected from C₁-C₆alkyl, for example C₁-C₄ alkyl such as methyl, C₁-C₆alkoxy, for example C₁-C₄ alkoxy such as methoxy, phenylethyl;
phenylpropyl;
phenyl(C=O)-(C₁ -C₆alkyl)-, for example phenyl(C=O)-(C₁ -C₄alkyl)-, such as -CH₂-(C=O)-Ph;
when R₃ is a 5, 6, or 7- membered, saturated or unsaturated, heterocyclic ring, as defined above, it may preferably be one of the following:
i) a 5-or 6-membered, saturated or unsaturated, heterocyclic ring directly attached to the quinazolinone ring;
ii) a 5-or 6-membered, saturated or unsaturated, heterocyclic ring attached to the quinazolinone ring via a methyl or ethyl linker;
iii) a 5-or 6-membered, saturated or unsaturated, heterocyclic ring directly attached to the quinazolinone ring or attached to the quinazolinone ring via a methyl or ethyl linker, containing one or two heteroatoms selected from N, O and S;
iv) any of i)-iii) above substituted with a substituent selected from cyano, C₁ -C₆alkyl, for example C₁ -C₄alkyl, such as ethyl or, particularly, methyl, halo, for example fluoro or, particularly, chloro, halo phenyl, for example fluoro- or, particularly, chlorophenyl; R₉-O-(C=O)-, for example C(O)OMe or, particularly, C(O)OEt, or =O;
v) any of i)-iv) above wherein the 5-or 6-membered, saturated or unsaturated, heterocyclic ring is selected from pyridine, furanyl, isoxazole, pyrrolidone, imidazole, thiophene, morpholine, pyrazine, pyrrole, piperidine and thiazole;
when R₃ is a 9- or 10- membered aromatic or heterocyclic fused ring as described above, it may preferably be one of the following:
i) a 9- or 10- membered aromatic or heterocyclic fused ring having zero, one or two heteroatons selected from N, O and S;
ii) a 9- or 10- membered aromatic or heterocyclic fused ring according to i) directly attached to the quinazolinone ring;
iii) a 9- or 10- membered aromatic or heterocyclic fused ring according to i) attached to the quinazolinone ring via a methyl or ethyl linker;
iv) a 9- or 10- membered aromatic or heterocyclic fused ring according to ii) or iii) optionally substituted with a substituent selected from halo, for example fluoro or, preferably, chloro, or hydroxyl;
v) a 9- or 10- membered aromatic or heterocyclic fused ring according to ii), iii) or iv), selected from naphthalene, benzothiazole, benzodioxole and quinoline;
and when R₃ is selected from group (a'), it is preferably phenyl substituted by chloro, bromo, C₁-C₄alkyl, hydroxy, C₁-C₄alkoxy or (C₃-C₆cycloalkyl)C₁-C₄alkoxy;
R₅ is most preferably hydrogen or hydroxyl.
R₆ is most preferably hydrogen or hydroxyl;
R12 is preferably hydrogen, formyl, C₁-C₆alkylcarbonyl or benzyl, the phenyl group of which is optionally substituted by 1, 2 or 3 substituents, selected from the group consisting of halogen, C₁-C₆alkyl, halo-C₁-C₆alkyl, hydroxy, hydroxy-C₁-C₆alkyl, C₁-C₆alkoxy, C₁-C₆alkoxy-C₁-C₆alkyl, halo-C₁-C₆alkoxy, C₁-C₆alkylthio, halo-C₁-C₆alkylthio, C₁-C₆alkylsulfinyl, halo-C₁-C₆alkylsulfinyl, C₁-C₆alkylsulfonyl, halo-C₁-C₆alkylsulfonyl, C₃-C₆cycloalkyl, C₃-C₆cycloalkyl-C₁-C₆alkyl, C₃-C₆cycloalkoxy, C₃-C₆cycloalkoxy-C₁-C₆alkyl, amino, C₁-C₆alkylamino, di-(C₁-C₆alkyl)amino, C₁-C₆alkoxycarbonylamino, cyano, formyl and C₁-C₆alkylcarbonyl, or is substituted at two adjacent carbon atoms by -O-CH₂-O- or -O-CF₂-O-,
preferably hydrogen, formyl, C₁-C₆alkylcarbonyl or benzyl,
more preferably hydrogen or formyl,
preferably hydrogen;
R13 and R8, taken together, may preferably represent, together with the three-membered moiety -N-C-C-, to which they are attached, a five-, six-, seven- or eight-membered, partially or fully unsaturated, optionally substituted, heterocyclic ring, which contains 1 ring nitrogen atom and optionally either 1 further ring nitrogen, oxygen or sulfur atom or 2 further ring nitrogen atoms, in which heterocyclic ring each ring oxygen or sulfur atom is bonded to 2 ring carbon atoms, the optional substituents of the said heterocyclic ring being selected from the group consisting of halogen, C₁-C₆alkyl, halo-C₁-C₆alkyl, hydroxy-C₁-C₆alkyl, C(O)-C₁-C₆alkyl and oxo, or R13 and R8, taken together, represent, together with the three-membered moiety - N-C-C-, to which they are attached, a five-, six- or seven-membered, partially or fully unsaturated, optionally substituted, heterocyclic ring, which contains 1 ring nitrogen atom and optional either 1 further ring nitrogen, oxygen or sulfur atom or 2 further ring nitrogen atoms, in which heterocyclic ring each ring oxygen or sulfur atom is bonded to 2 ring carbon atoms, the optional substituents of the said heterocyclic ring being selected from the group consisting of halogen, C₁-C₆alkyl, C(O)-C₁-C₆alkyl, halo-C₁-C₆alkyl, hydroxy-C₁-C₆alkyl and oxo, or
N-R13 and R8-, taken together, represent a moiety N-X-O- (Ibb), in which -X- is - C(=O)- or -(CH₂)ₐ-, in which a is 2 or 3 and in which any methylene group, independently from any other methylene group in the moiety Ibb, is, optionally, monosubstituted by oxo or substituted by 1 or 2 substituents, selected from the group consisting of halogen, C₁-C₆alkyl, C(O)-C₁-C₆alkyl, halo-C₁-C₆alkyl and hydroxy-C₁-C₆alkyl, or N-R13 and R8-, taken together, represent a moiety N-C(Rₐ)=C(R_{b})- (Idd), in which Rₐ is hydrogen, C₁-C₆alkyl, halo-C₁-C₆alkyl or hydroxy-C₁-C₆alkyl and R_{b} is hydrogen, C₁-C₆alkyl, halo-C₁-C₆alkyl or hydroxy-C₁-C₆alkyl, or N-R13 and R8-, taken together, represent a moiety N-C(R_{c})=N- (lee), in which R_{c} is hydrogen, C₁-C₆alkyl or halo-C₁-C₆alkyl, or N-R13 and R8-, taken together, represent a moiety N-N=C(R_{d})-(Iff), in which R_{d} is hydrogen, C₁-C₆alkyl or halo-C₁-C₆alkyl, or N-R13 and R8-, taken together, represent a moiety N-N=C(R_{f})- (Ig), in which R_{f} is halogen, or N-R13 and R8-, taken together, represent a moiety N-N=N- (Ih) or N-R13 and R8-, taken together, represent a moiety N-(CH₂)₂-N(H)-C(R_{g})H- (Iii), in which R_{g} is hydrogen, C₁-C₆alkyl or halo-C₁-C₆alkyl,
preferably N-R13 and R8-, taken together, represent a moiety N-X-O- (Ibb), in which - X- is -C(=O)- or -(CH₂)ₐ-, in which a is 2 or 3 and in which any methylene group, independently from any other methylene group in the moiety Ibb, is optionally substituted by 1 or 2 substituents, selected from the group consisting of C₁-C₆alkyl and hydroxy-C₁-C₆alkyl, or N-R13 and R8-, taken together, represent a moiety N-C(Rₐ)=C(R_{b})- (Idd), in which Rₐ is hydrogen and R_{b} is C₁-C₆alkyl, or N-R13 and R8-, taken together, represent a moiety N-C(R_{c})=N- (lee), in which R_{c} is hydrogen, C₁-C₆alkyl or halo-C₁-C₆alkyl, or N-R13 and R8-, taken together, represent a moiety N-N=C(R_{d})- (Iff), in which R_{d} is hydrogen or C₁-C₆alkyl, or N-R13 and R8-, taken together, represent a moiety N-N=C(R_{f})- (Ig), in which R_{f} is halogen, or N-R13 and R8-, taken together, represent a moiety N-N=N- (Ih) or N-R13 and R8-, taken together, represent a moiety N-(CH₂)₂-N(H)-C(R_{g})H- (Iii), in which R_{g} is hydrogen.

"C₁-C₈alkyl" denotes straight-chain or branched C₁ to C₆-alkyl; "C₁-C₆alkyl" denotes straight-chain or branched C₁ to C₆-alkyl; and "C₁-C₄alkyl" denotes straight-chain or branched C₁ to C₆-alkyl;e.g., methyl ethyl, *n*-propyl, isopropyl, *n*-butyl, isobutyl, *sec*-butyl or *tert*-butyl.

"C₂-C₆alkenyl" denotes straight-chain or branched C₂ to C₆-alkenyl, e.g., ethenyl, *n*-propenyl or isopropenyl.

"C₁-C₆alkoxy" denotes straight-chain or branched C₁ to C₆-alkyl-oxy, e.g., methoxy, ethoxy, *n*-propoxy or isopropoxy.

"Halo" denotes halogen which may be I, Br, Cl or F.

"Esterified hydroxy" denotes acyloxy, preferably C₁-C₆alkanoyloxy, more preferably C₁-C₄alkanoyloxy.

"Etherified hydroxy" denotes C₁-C₆alkoxy, preferably C₁-C4alkoxy.

The quinazolinone compounds of the invention exist in free or salt form. The invention is to be understood as including the compounds of formula (I) in free or salt form. The present invention also relates to a process for the preparation of a compound of formula I, in free form or in salt form, according to the following representative reaction schemes, in which "Ar" denotes R₃ as defined above.

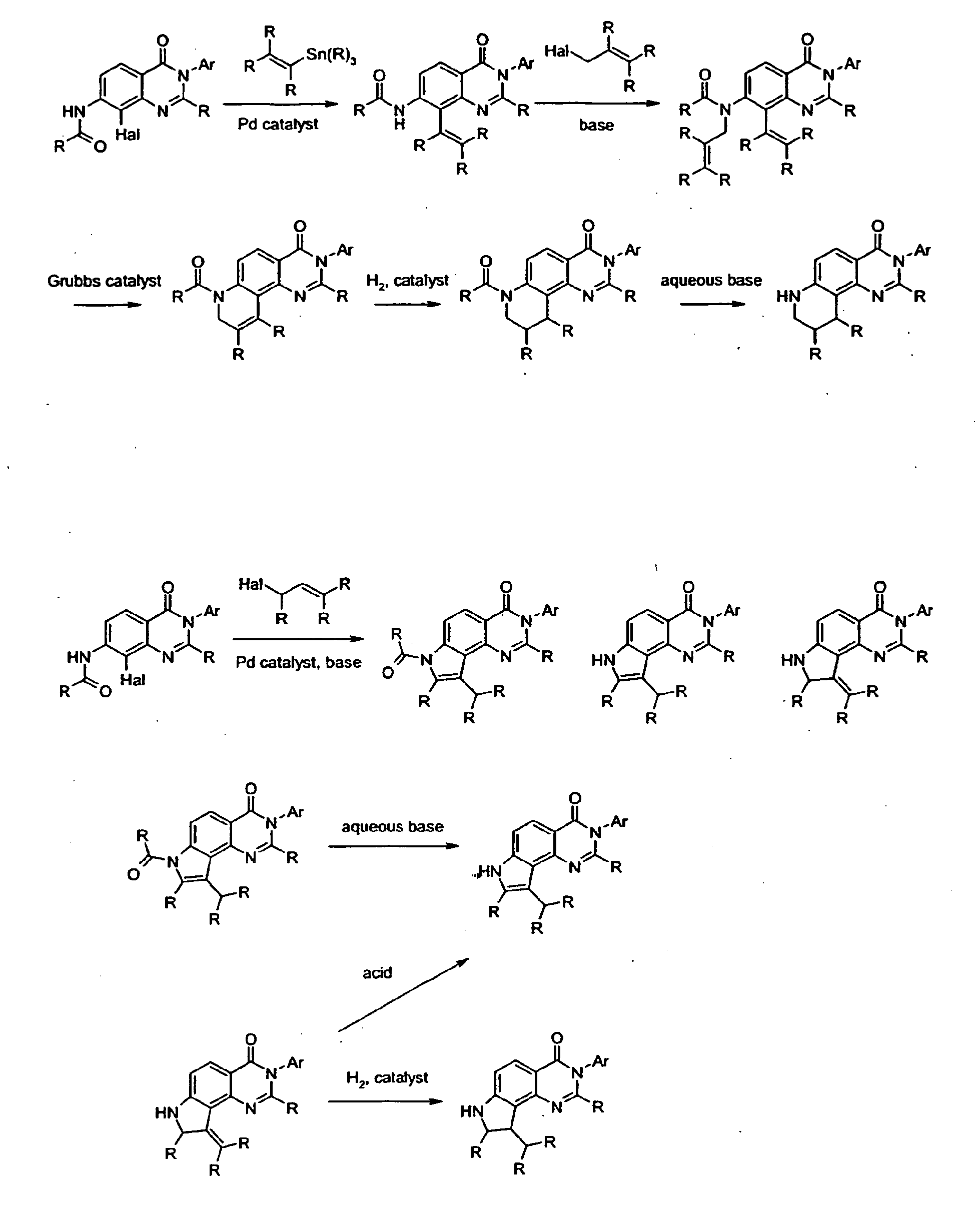

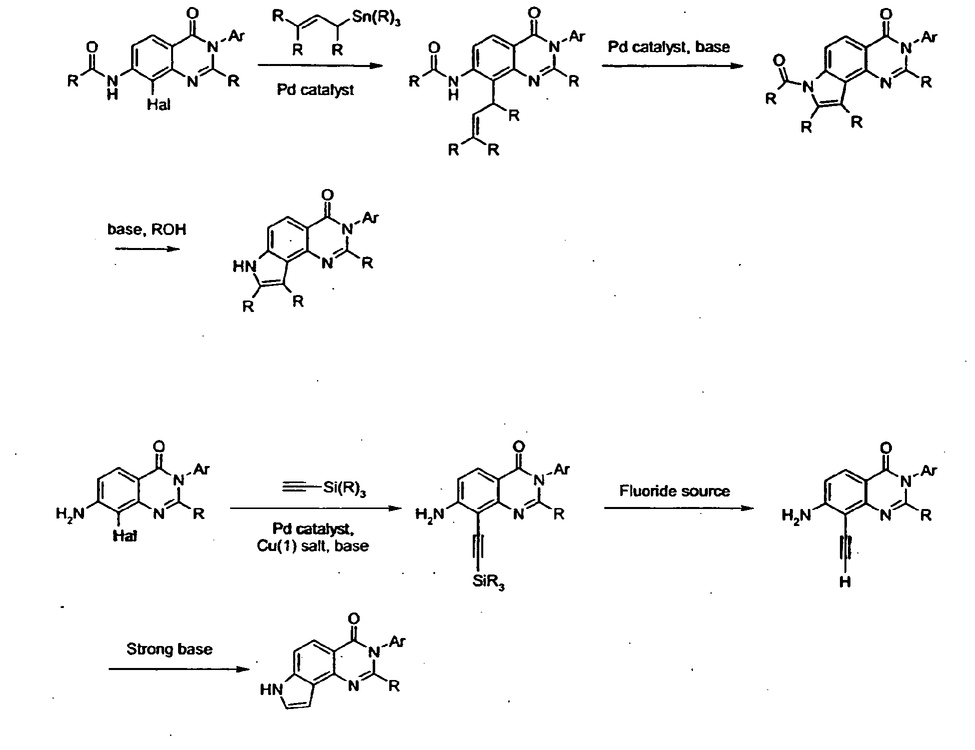

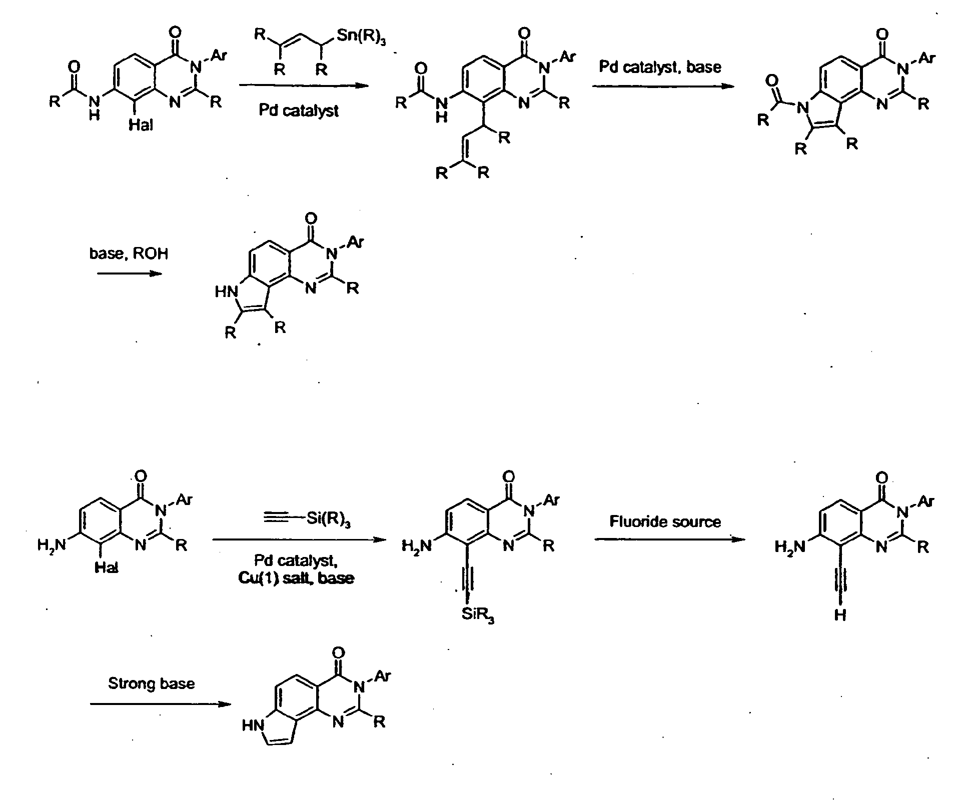

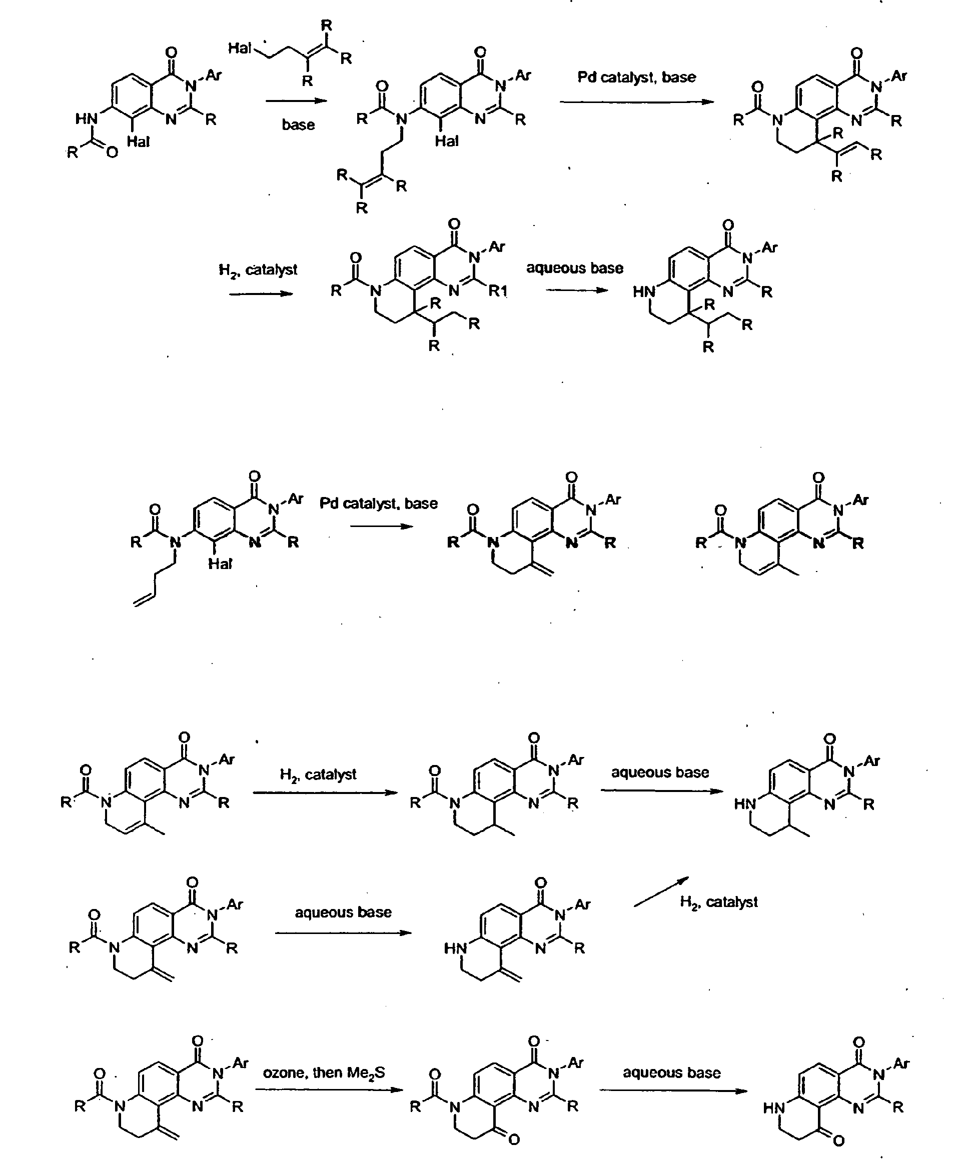

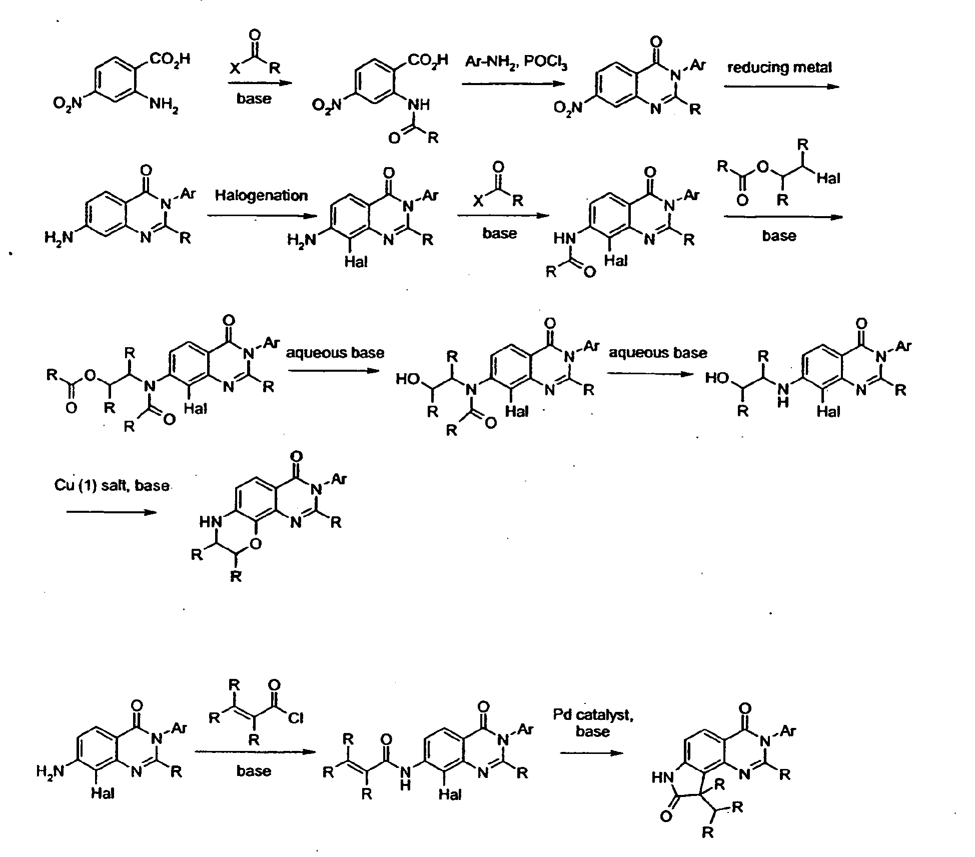

In the above schemes, R can be alkyl or another suitable group.

Detailed reaction conditions are described in the Examples.

The working-up of the reaction mixtures and the purification of the compounds thus obtainable may be carried out in accordance with known procedures.

Acid addition salts may be produced from the free bases in known manner, and vice-versa.

Compounds of formula (I) in optically pure form can be obtained from the corresponding racemates according to well-known procedures, e.g., HPLC with chiral matrix. Alternatively, optically pure starting materials can be used.

Stereoisomeric mixtures, e.g., mixtures of diastereomers, can be separated into their corresponding isomers in a manner known per se by means of suitable separation methods. Diastereomeric mixtures, e.g., may be separated into their individual diastereomers by means of fractionated crystallisation, chromatography, solvent distribution and similar procedures. This separation may take place either at the level of a starting compound or in a compound of formula (I) itself. Enantiomers may be separated through the formation of diastereomeric salts, e.g., by salt formation with an enantiomer-pure chiral acid, or by means of chromatography, e.g., by HPLC, using chromatographic substrates with chiral ligands.

In any additional process steps, carried out as desired, functional groups of the starting compounds which should not take part in the reaction may be present in unprotected form or may be protected, e.g., by one or more of the protecting groups mentioned below. The protecting groups are then wholly- or partly-removed according to one of the methods described there.

The protecting groups may already be present in precursors and should protect the functional groups concerned against unwanted secondary reactions. It is a characteristic of protecting groups that they lend themselves readily, i.e., without undesired secondary reactions, to removal, typically by solvolysis, reduction, photolysis or also by enzyme activity, e.g., under conditions analogous to physiological conditions, and that they are not present in the end-products. The skilled artisan knows, or can easily establish, which protecting groups are suitable with the reactions mentioned hereinabove and hereinafter.

The protection of such functional groups by protecting groups, the protecting groups themselves, and their removal reactions are described, e.g., in standard reference works, such as J.F.W. McOmie, Protective Groups in Organic Chemistry, Plenum Press, London and NY (1973); T.W. Greene, Protective Groups in Organic Synthesis, Wiley, NY (1981); The Peptides; Volume 3, E. Gross and J. Meienhofer, Eds., Academic Press, London and NY (1981); Methoden der organischen Chemie (Methods of organic chemistry), Houben Weyl, 4th Edition, Volume 15/1, Georg Thieme Verlag, Stuttgart (1974); H.D. Jakubke and H. Jescheit, Aminosauren, Peptide, Proteine (Amino acids, peptides, proteins), Verlag Chemie, Weinheim, Deerfield Beach, and Basel (1982); and Jochen Lehmann, Chemie der Kohlenhydrate: Monosaccharide und Derivate (Chemistry of carbohydrates: monosaccharides and derivatives), Georg Thieme Verlag., Stuttgart (1974).

All process steps described herein can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralizing agents, e.g., ion exchangers, typically cation exchangers, e.g., in the H⁺ form, depending on the type of reaction and/or reactants at reduced, normal or elevated temperature, e.g., in the range from -100°C to about 190°C, preferably from about -80°C to about 150°C, e.g., at -80°C to 60°C, at room temperature, at -20°C to 40°C or at the boiling point of the solvent used, under atmospheric pressure or in a closed vessel, where appropriate under pressure, and/or in an inert atmosphere, e.g., under argon or nitrogen.

Another aspect of this invention relates to the fact that the compounds of formulae (I) and and their pharmaceutically acceptable salts, have beneficial pharmacological activity and, therefore, are useful as pharmaceuticals. In particular, the compounds of formula (I)exhibit human vanilloid antagonistic activity. More particularly, the compounds of formula (I)are active at the TRPVI receptor as demonstrated by their ability to inhibit capsaicin and/or low pH activation of the TRPVI ion channel as follows:

Chinese Hamster Ovary-K1 (CHO-K1) cells, transfected to express either the human, rat or guinea pig TRPV1 receptor, are grown in Minimal Essential Media (MEM) alpha medium without nucleosides supplemented with fetal calf serum (10%), 2 mM L-glutamine, 100 IU/mL penicillin, 100 µg/mL streptomycin and 350-700 µg/mL geneticin. All reagents are supplied by Invitrogen. Cells are grown in T-175 flasks or clear bottom 96- or 384-well plates and maintained at 37°C in a 90% humidified incubator with an atmosphere of 5% CO₂ and 95% air. The cells are passaged twice a week and for experimentation, cells are harvested at approximately 80% confluency and plated onto view plates at 35,000-40,000 cells per well in 100 µL media and grown overnight.

### Calcium mobilisation assay

On the day of the assay, media is aspirated and cells are washed with 10 mM *N*-2-(hydroxyethylpiperazine-*N*'-[2-ethane-sulfonic acid] (HEPES) buffered Hank's Balanced Salt Solution (HBSS), pH 7.4. Cells are then incubated with a fluorescent sensitive-calcium binding dye, typically fluo-4/AM (from Molecular Probes), made up in HEPES buffered HBSS, containing pluronic F-127 with or without probenicid. For the pH assay, HEPES is omitted and the pH of HBSS adjusted to 7.4. After washing, cells are incubated with test compounds (made up in HBSS, pH 7.4), in duplicate. The TRPV1 receptor is stimulated by addition of either capsaicin at an approximate EC₈₀ concentration or a low pH buffered solution [60 mM 2-[*N*-morpholino] ethane sulfonic acid (MES) in HBSS] to give a final pH of 5.5. The cellular responses are followed in fluorescent plate reader, typically a Molecular Devices Flexstation. The response in the presence of the antagonist is calculated as a percentage of the control response to capsaicin or low pH and is plotted against the concentration of antagonist. The IC₅₀ values (concentrations of antagonist that inhibit responses to either pH 5.5 or capsaicin by 50%) is estimated by non-linear regression analysis to sigmoidal-logistic curves. These values are averaged (means and standard error of the mean) for at least three independent experiments.

A specific example of a calcium mobilization assay is as follows:

On the day of the capsaicin assay, media is aspirated and cells are washed with 100 µL 10 mM *N*-2-(hydroxyethylpiperazine-*N*'-[2-ethane-sulfonic acid] (HEPES) buffered Hank's Balanced Salt Solution (HBSS), pH 7.4. Cells are then incubated for 40-60 minutes with 2.3 µM of the calcium binding dye fluo-4/AM (from Molecular Probes), made up in HEPES buffered HBSS, containing 0.01% pluronic F-127 and 2mM probenicid. For the pH assay, HEPES is omitted and the pH of HBSS adjusted to 7.4. After washing twice with 100 µL assay buffer, cells are incubated for 10 minutes with 100 µL of test compounds (made up in HBSS, pH 7.4), in duplicate. The plate is then placed in a Molecular Devices Flexstation. The TRPV1 receptor is stimulated by application of either capsaicin or low pH. For testing the effect of compounds for possible antagonism, capsaicin is used at an approximate EC₈₀ concentration of 0.05 µM. For pH experiments, a low pH buffered solution [60 mM 2-[*N*-morpholino] ethane sulfonic acid (MES) in HBSS] is added to the assay wells to give a final pH of 5.5.

For determinations of antagonist IC₅₀ values (concentrations of antagonist that inhibit responses to either pH 5.5 or capsacin by 50%), at least 10 antagonist concentrations are measured in duplicate. The response in the presence of the antagonist is calculated as a percentage of the control response to capsaicin or low pH and is plotted against the concentration of antagonist. The IC₅₀ is estimated by non-linear regression analysis to sigmoidal-logistic curves by Activity-Base software (v5.0.10) or Microcal Origin (v7.03). These values are averaged (means and standard error of the mean) for at least three independent experiments.
The agents of the invention are useful in the prevention and treatment of diseases and conditions in which human VR1 activation plays a role or is implicated, and therefore susceptible to treatment by the modulation (preferably antagonism) of VR1 receptors. Such conditions include, in particular, acute or chronic pain of somatic or visceral origin, inflammatory or obstructive airways disease, urinary incontinence or overactive bladder, inflammatory skin diseases, inflammatory disorders of the gastrointestinal tract, diabetes, obesity and obesity-related diseases, psychiatric disorders, and treatment of the consequences exposure to VR1 agonists.

The agents of the invention are particularly useful in the treatment or prevention of chronic pain with an inflammatory component such as rheumatoid arthritis; bone and joint pain (osteoarthritis); post-surgical or trauma pain including dental pain e.g. following third molar extraction, post mastectomy pain and pain associated with sprains or fractures; musculo-skeletal pain such as fibromyalgia; myofascial pain syndromes; headache, including migraine, acute or chronic tension headache, cluster headache, temporomandibular pain, and maxillary sinus pain; ear pain; episiotomy pain; bums, and especially primary hyperalgesia associated therewith; deep and visceral pain, such as heart pain, muscle pain, eye pain, orofacial pain, abdominal pain, gynaecological pain, such as dysmenorrhoea, and labour pain; hemorrhoids; pain associated with the urogenital tract such as cystitis and vulvadynia; chronic pain associated with nerve injury and/or diseases affecting the nervous system, such as neuropathic pain associated with post-herpetic neuralgia, diabetic neuropathy, chemotherapy-induced neuropathy, amputations ("phantom limb pain"), nerve entrapment and brachial plexus avulsions, low back pain, sciatica and ankylosing spondylitis, reflex sympathetic dystrophy and other chronic nerve injuries; complex regional pain syndromes; Glossodynia or burning mouth syndrome; central nervous system pain, such as pain due to spinal cord or brain stem damage, multiple sclerosis or stroke; gout; scar pain; pain associated with carcinoma, often referred to as cancer pain; pain associated with viral (e.g. HIV)-induced neuropathy, alcohol and narcotic abuse; pain and other symptoms associated with sun or UV burn, exposure to VR1 agonist (e.g. capsaicin, acid, tear gas, noxious heat or pepper spray), snake, spider or insect bite and jellyfish sting.

Gastrointestinal disorders to be treated in accordance with the invention include those associated with gastrointestinal hypersensitivity, visceral pain and/ or altered motor responses (including electrolyte/water secretion) such as functional bowel disorders and functional gastrointestinal disorders, including irritable bowel syndrome (IBS), functional dyspepsia, heartburn, non-erosive reflux disease, intestinal pseudoobstruction, functional abdominal bloating, and functional abdominal pain; other conditions associated with visceral hypersensitivity including gastro-oesophageal reflux disease and emesis, oesophagitis, post-operative visceral pain, post-operative ileus, visceral smooth muscle spasms, ulcerative colitis, Crohn's disease, ulcers, chronic constipation, diarrhea, early satiety, epigastric pain, nausea, vomiting, burbulence, anal incontinence, faecal urgency and rectal hypersensitivity, gastroparesis, e. g. diabetic gastroparesis, pancreatitis and Hirschsprung's disease.

Urinary incontinence ("UI") or overactive bladder to be treated in accordance with the invention is a broad term that covers a range of disorders and symptoms including urge UI, stress UI, mixed urge/stress UI, neurogenic UI, bladder detrusor hyperreflexia (neurogenic detrusor overactivity), detrusor instability (idiopathic detrusor overactivity), decreased bladder compliance, weakness of urethal sphincter, urinary outlet obstruction, interstitial cystitis, nephritis, uveitis, sensory urgency, motor urgency, nocturia, and bladder-related visceral pain.

The agents of the invention are also useful as agents for the therapy of hyperreactive, inflammatory or obstructive airways diseases including asthma, inflammatory airways disease, e.g. chronic obstructive pulmonary or airways disease (COPD or COAD), adult respiratory distress syndrome (ARDS), chronic bronchitis, pneumoconiosis, e.g. aluminosis, anthracosis, asbestosis, chalicosis, ptilosis, siderosis, silicosis, tabacosis, byssinosis; rhinitis including allergic rhinitis such as seasonal and perennial rhinitis, and non-allergic rhinitis; cough, either idiopathic or associated with respiratory diseases such as COPD, asthma, cystic fibrosis, cancer, or gastrointestinal disturbances such as gastro-oesophageal reflux.

The agents of the invention may also have therapeutic benefit in inflammatory skin disorders, for example psoriasis and eczema, or itch of non-specific origin; contact dermatitis and hypersensitivity; autoimmune or inflammatory diseases, including Crohn's disease, ulcerative colitis and Gullian Barre Syndrome; multiple chemical sensitivity, neurological diseases like anxiety, panic disorders, depression, schizophrenia, cognition, Parkinson's Disease and Alzheimer's Disease; hair loss; diabetes; obesity and obesity-related diseases; as anti-spasmodics, e.g. for the treatment of spasm of the gastrointestinal tract or uterus; for the therapy of septic shock, e.g. as anti-hypovolaemic and / or anti hypotensive agents; cerebral oedema.

For the above-mentioned indications, the appropriate dosage will of course vary depending upon, e.g., the compound employed, the host, the mode of administration and the nature and severity of the condition being treated. However, in general, satisfactory results in animals are indicated to be obtained at a daily dosage of from about 0.05 to about 150, preferably from about 0.1 mg/kg to about 100 mg/kg animal body weight. In larger mammals, e.g., humans, an indicated daily dosage is in the range from about 0.5 to about 5,000, preferably from about 1 mg to about 500 mg of a compound of formula (I), conveniently administered, e.g., in divided doses up to four times a day or in sustained-release form.

The agents of the invention can be administered in vivo either alone or in combination with other pharmaceutical agents, e.g. agents effective in the treatment of diseases and conditions in which the human VR1 activation plays a role or is implicated. A suitable combination consists of a compound of the present invention with a compound selected from the class or individuals from the following list: Dopamine D₂ antagonists, eg domperidone, metoclopramide and itopride; 5HT₄ receptor agonists, eg cisapride, cinitapride, mosapride, renzapride, prucalopride, tegaserod, and compounds described in WO 2005068461 (Aryx), e.g. AT-7505, US 2005228014 and WO 2005080389 (Theravance), e.g. TDI-2749, US 2006100426, US 2006100236, US 2006135764, US 20060183901, WO 200610827, WO 2006094063, WO 2006090224, WO2006090279, US 2005277671, WO 2005092882, WO 2005073222, JP 2005104896, JP 2005082508, WO 2005021539, JP 2004277319, JP 2004277318, WO 2004026869 and EP 1362857;
5HT₃ agonists, eg pumosetrag;
CCK_{A} receptor antagonists, eg loxiglumide and dexloxiglumide;
Motilin receptor agonists, eg motilin, atilmotilin, erythromycin, alemcinal, mitemcinal, KOS-2187 and compounds described in WO 2005060693;
µ-opioid antagonists, eg alvimopan and methylnaltrexone;
Opioid agonists, eg asimadoline, loperamide and codeine;
CRF-1 receptor antagonists, eg GSK876008 and compounds described in WO 2004069257, WO 9940089, US 6844351, WO 2005013997, WO 2005014557, WO 2005023806, WO 2005026126, WO 2005028480, WO 2005044793, WO 2005051954, WO 2005051954, WO 2005115399, WO 2005028480, WO 2005023806, WO 2006044958, US 20060211710 and WO 2006108698;
Glutamate receptor antagonists, eg AZD9272 and compounds described in WO 9902497, WO 2000020001, WO 200304758 and WO 2005030723;
Neurokinin receptor antagonists, eg casopitant, nepadutrent saredutant, DNK-333, SLV-317, SLV321, SLV317 and compounds described in EP 96-810237;
5HT₃ receptor antagonists, eg alosetron, cilansetron, ramosetron, azasetron, ondansetron, granisetron tropisetron and DDP225;
Histamine H₂ antagonists, eg famotidine, cimetidine, rantidine and nizatidine;
Histamine H₄ antagonists, eg JNJ7777120, JNJ10191584 and compounds described in US 2006111416, WO 2006050965, WO 2005092066, WO 2005054239 US 2005070550, US 2005070527 and EP 1505064;
Proton pump inhibitors, eg omeprazole, lansoprazole, rabeprazole, tentoprazole, pantoprazole, esomeprazole, revaprazan soraprazan and AGN201904;
Chloride channel activators, eg lubiprostone;
Guanylate cyclase activators, eg linaclotide;
Muscarinic antagonists, eg darifenacin, solifenacin, atropine, dicycloverine, hycosine butyl bromide, propantheline, oxybutinin, cimetropium bromide, pinaverium bromide and otilonium bromide;
Antispasmodics, eg mebeverine, tiropramide, alverine and peppermint oil;
Stimulant laxatives, eg bisacodyl;
Osmotic laxatives, eg activated charcoal with sorbitol, lactulose, magnesium hydroxide and phosphate buffered saline;
Faecal softeners, eg senna concentrate, liquid paraffin and arachis oil;
Absorbents and fibre supplements, eg bulk fibre laxatives such as bran, methycellulose, ispaghula husk and sterculia;
Antacids, eg aluminium, magnesium and calcium antacids, simeticone and alginate containing preparations;
GI relaxants, eg cholestyramine resin;
Bismuth compounds, eg bismuth subsalicylate;
Vanilloid receptor antagonists, eg compounds described in WO 2002076946, WO 2004033435, WO 2005121116 and WO 2005120510;
Anticonvulsants, eg carbamazepine, oxcarbemazepine, lamotrigine, gabapentin, and pregabalin;
NSAIDS, eg aspirin, acetometaphen, ibuprofen, diclofenac, naproxen, flurbiprofen, indomethacin, piricoxam, ketoprofen, sulindac and diflunisal;
COX-2 inhibitors eg celecoxib, rofecoxib, lumiracoxib, valdecoxib, etoricoxib and compounds described in WO 2004048314;
Opiates, eg morphine, buprenorphine, diamorphine; dihydrocodeine, fentanyl and pethidine;
GABA_{b} modulators, eg racemic and (R)-badofen, AZD3355, XP19986 and compounds described in WO 2006001750 and WO 2004000856;
CB receptor ligands, eg compounds described in WO 2002042248 and WO 2003066603;
Calcium channel blockers, eg ziconotide, AGI0-003, PD-217014 and compounds described in WO 2006038594, WO 2006030211 and WO 2005068448;
Sodium channel blockers, eg lamotrigine and compounds described in WO 2006023757, WO 2005097136, JP 2005206590 and WO 2005047270;
Tricyclic antidepressants, e.g. clomipramine, amoxapine, nortripyline, amitriptyline, imipramine, desipramine, doxepin, trimipramine and protripyline;
Selective serotonin reuptake inhibitors, eg fluoxetine, paroxetine, citaprolam, sertaline, fluvoxamine, duloxetine;
Anxiolytic agents, eg milnacipran, tianeptine, MCI-225 and dextofisopam;
CGRP antagonists, eg olcegepant and cizolirtine;
5HT_{1d} antagonists, eg almotriptan, eletriptan, frovatriptan, naratriptan, rizatriptan, sumatriptan and zolmatriptan; and
Bradykinin receptor antagonists, eg compounds described in WO 2000075107, WO 2002092556 and WO 20050851298.

The pharmaceutical compositions for separate administration of the combination partners and for the administration in a fixed combination, i.e., a single galenical composition comprising at least two combination partners, according to the invention can be prepared in a manner known per se and are those suitable for enteral, such as oral or rectal, and parenteral administration to mammals, including man, comprising a therapeutically effective amount of at least one pharmacologically active combination partner alone or in combination with one or more pharmaceutically acceptable carriers, especially suitable for enteral or parenteral application.

Pharmaceutical compositions contain, e.g., from about 0.1% to about 99.9%, preferably from about 20% to about 60%, of the active ingredients. Pharmaceutical preparations for the combination therapy for enteral or parenteral administration are, e.g., those in unit dosage forms, such as tablets including sugar-coated tablets, capsules, suppositories and ampoules. These are prepared in a manner known, per se, e.g., by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

A further aspect of the instant invention involves the novel compositions comprising a pharmaceutically acceptable carrier or diluent and a therapeutically effective amount of a compound of formula (I), in free or salt form.

In accordance with the foregoing, the present invention also provides:
(1) A compound of formula (I) in free or salt form for use as a vanilloid receptor blocker, e.g., for use in any of the particular indications set forth hereinabove;
(2) A compound of formula (I) in free or salt form for the treatment of a disease or condition in which vanilloid receptor plays a role or is implicated;
(3) Use of a compound of formula (I) in free or salt form for the manufacture of a medicament for the treatment or prevention of a disease or condition in which activity of vanilloid receptor plays a role or is implicated;
(4) A method as set forth hereinabove comprising co-administration, e.g., concomitantly or in sequence, of a therapeutically effective amount of a vanilloid receptor antagonist, e.g., a compound of formula (I) in free or salt form and a second drug substance, said second drug substance being, e.g., for use in any of the particular indications set forth hereinabove; and
(5) A combination comprising a therapeutically effective amount of a compound of formula (I) in free or salt form and a second drug substance, said second drug substance being, e.g., for use in any of the particular indications set forth hereinabove.

### Examples

In the Examples which follow, which are not intended to limit, in any way, the scope of the present invention, the following abbreviations are used:

| | |
|---|---|
| eq. | equivalent(s) |
| h | hour(s) |
| min | minute(s) |

The HPLC retention time (RT) data correspond to the following conditions:
Phenomenex Luna reversed phase C18 3 micron (30 x 4.6 mm) column; column temperature 25°C; gradient elution 10 % MeCN in water (+ 0.08 % formic acid) to 100 % MeCN over 10 minutes (rate = 3.0 ml/minute). The purity values are quoted at 254 nm.

### Preparative Examples

### 3-(4-chlorophenyl)-2-isopropyl-7-nitro-3H-quinazolin-4-one

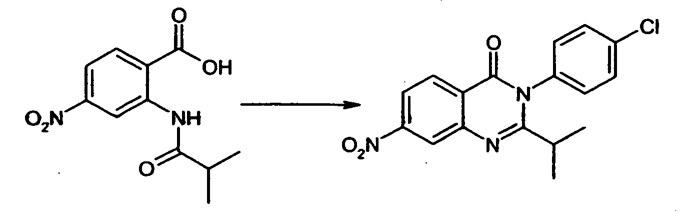

**A suspension of 4-nitroanthranilic acid isobutyramide (4-g, 15.8 mmol), 4-**chloroaniline (2.2 g, 17.2 mmol) and phosphorus trichloride ( 5.6 mL) in toluene (150 mL) was heated to reflux (bath temperature 150 °C) for 2 h. The reaction mixture was allowed to cool to room temperature and then evaporated to dryness. The residue was partitioned between water and EtOAc and the aqueous phase was extracted (x2) with EtOAc. The combined organic phases were washed with water, dried (Na₂SO₄) and evaporated *in vacuo.* Trituration with isopropyl ether provided the title compound as a brown solid (4.2 g, 77%).

### 7-Amino-3-(4-chlorophenyl)-2-isopropyl-3H-quinazolin-4-one

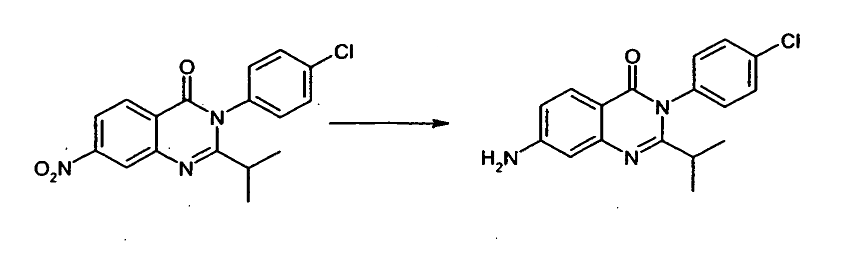

A mixture of 3-(4-chlorophenyl)-2-isopropyl-7-nitro-3H-quinazolin-4-one (2.4 g, 6.98 mmol), iron powder (1.16 g, 20.8 mmol) and glacial acetic acid (70 mL) was stirred at 50 °C for 2.5 h. The reaction mixture was allowed to cool to room tremperature and then evaporated *in vacuo* to dryness. The residue was partitioned between water and EtOAc and the aqueous phase was extracted (x2) with EtOAc. The combined organic phases were washed with water, dried (Na₂SO₄) and evaporated *in vacuo* to give a brown solid. Purification by automated flash chromatography (gradient elution: EtOAc/DCM 0 to 50%) provided the title compound as a pale yellow solid (1.74 g, 79%).

### 7-Amino-8-bromo-3-(4-chloro-phenyl)-2-isopropyl-3H-quinazolin-4-one

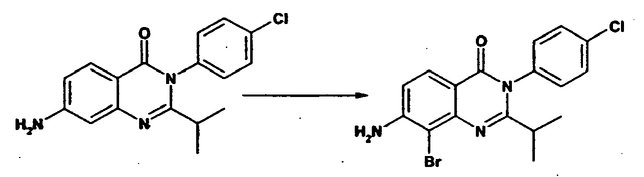

To a solution of 7-Amino-3-(4-chloro-phenyl)-2-isopropyl-3H-quinazolin-4-one (1.0 g, 3.2 mmol) in chloroform (20 ml) was added N-bromosuccinimide. After stirring for 5 minutes at ambient temperature TLC indicated complete conversion. The reaction mixture was diluted with water and dichloromethane, and the aqueous layer was extracted with dichloromethane. The combined aqueous extracts were dried over anhydrous sodium sulfate and evaporated to give a dark brown solid. The crude product was triturated with diethyl ether to provide the title compound as a grey solid, which was recovered by filtration and dried (1.02 g, 2.6 mmol, 81%).

### 7-Amino-8-iodo-3-(4-chloro-phenyl)-2-isopropyl-3H-quinazolin-4-one

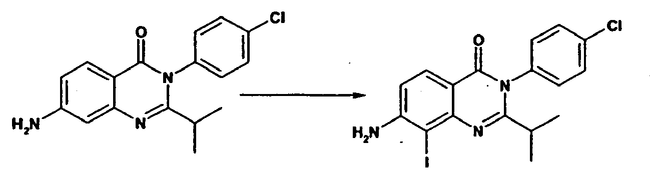

The title compound was prepared in an analogous manner to **7-Amino-8-bromo-3-(4-chloro-phenyl)-2-isopropyl-3H-quinazolin-4-one,** using N-iodosuccinimide instead of N-bromosuccinimide and extending the reaction time to 1.5 h. Purification by automated gradient elution flash chromatography (eluent hexane to hexane/ethyl acetate 7/3) provided the title compound as a pale brown solid, 94%.

### N-[3-(4-Chloro-phenyl)-8-iodo-2-isopropyl-4-oxo-3,4-dihydro-quinazolin-7-yl]-acetamide

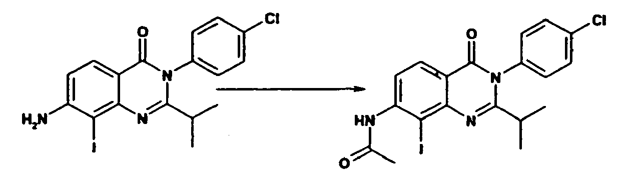

To a solution of 7-Amino-8-iodo-3-(4-chloro-phenyl)-2-isopropyl-3H-quinazolin-4-one (300 mg, 0.682 mmol) in THF/acetic anhydride 20/1 (21 ml) was added concentrated hydrochloric acid ( 3 drops). The reaction mixture was stirred at room temperature overnight and TLC/LCMS analysis indicated complete conversion. The reaction mixture was diluted with water and extracted with ethyl acetate (x 2). The combined organic extracts were dried over anhydrous sodium sulfate and evaporated to provide the title compound as an off-white solid (320 mg, 0.664 mmol, 97%), which was used without further purification.

### N-[8-Bromo-3-(4-chlorophenyl)-2-isopropyl-4-oxo-3,4-dihydroquinazolin-7-yl]-acetamide

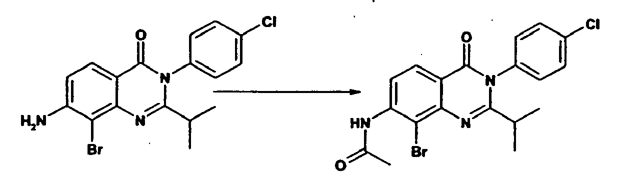

The title compound was prepared in an analogous manner to **N-[3-(4-Chlorophenyl)-8-iodo-2-isopropyl-4-oxo-3,4-dihydro-quinazolin-7-yl]-acetamide.** Yield: 97%

### Acetic acid 2-{acetyl-[3-(4-chloro-phenyl)-8-iodo-2-isopropyl-4-oxo-3,4-dihydro-quinazolin-7-yl]-amino}-ethyl ester

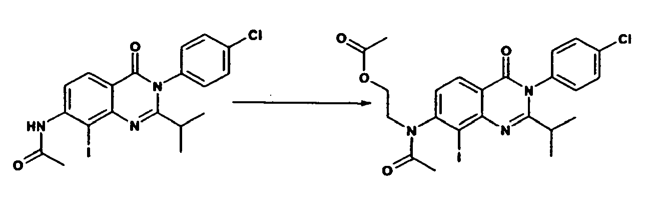

To a solution of N-[3-(4-Chloro-phenyl)-8-iodo-2-isopropyl-4-oxo-3,4-dihydro-quinazolin-7-yl]-acetamide (300 mg, 0.622 mmol) in DMF was added sodium hydride (37 mg, 60% dispersion in mineral oil, 0.933 mmol) and the mixture was stirred at ambient temperature for 20 min. 2-Bromoethyl acetate (0.066 ml, 0.933 mmol) was added and stirring was continued overnight. TLC and LCMS analysis indicated incomplete conversion. Cesium carbonate (202 mg, 0.622 mmol), additional 2-bromoethyl acetate (0.044 ml, 0.622 mmol) and potassium iodide (catalytic) were added and stirring was continued for 4 h. TLC and LCMS analysis indicated a trace of starting material remained. The reaction mixture was diluted with water and extracted with ethyl acetate (x 3). The combined organic extracts were dried over anhydrous sodium sulfate and evaporated. Purification by automated gradient elution flash chromatography (eluent hexane to ethyl acetate) provided the title compound 326 mg, 0.574 mmol, 92%.

### N-[3-(4-Chloro-phenyl)-8-iodo-2-isopropyl-4-oxo-3,4-dihydro-quinazolin-7-yl]-N-(2-hydroxyethyl)acetamide

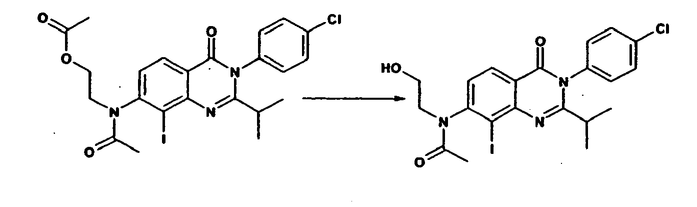

To a solution of acetic acid 2-{acetyl-[3-(4-chloro-phenyl)-8-iodo-2-isopropyl-4-oxo-3,4-dihydro-quinazolin-7-yl]-amino}-ethyl ester (300 mg, 0528 mmol) in methanol/water 2/1 (6 ml) was added potassium carbonate (88 mg, 0.634 mmol) and the reaction mixture was stirred at ambient temperature for 20 min. LCMS analysis indicated completion of the reaction.. The reaction mixture was diluted with water and extracted with dichloromethane (x 2). The combined organic extracts were dried over anhydrous sodium sulfate and evaporated to provide the title compound as a foam, 271 mg, 0.516 mmol, 97%.

### 3-(4-Chloro-phenyl)-7-(2-hydroxyethylamino)-8-iodo-2-isopropyl-3H-quinazolin-4-one

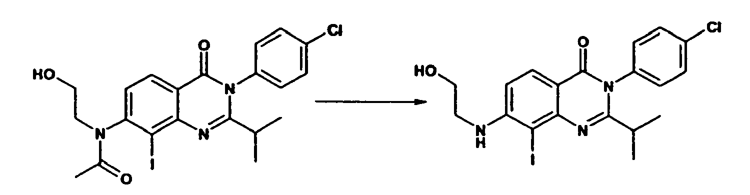

To a solution of N-[3-(4-Chloro-phenyl)-8-iodo-2-isopropyl-4-oxo-3,4-dihydro-quinazolin-7-yl]-N-(2-hydroxyethyl)acetamide (135 mg, 0.257 mmol) in methanol (2 ml) was added %M potassium hydroxide solution ( 1 ml). the reaction mixture was stirred at ambient temperature for 10 min and then heated to 50 °C on an oil bath for 20 min. TLC and LCMS analysis indicated complete conversion. The reaction mixture was acidified with dilute hydrochloric acid and extracted with ethyl axcetate (x 2). The combined organic extracts were dried over anhydrous sodium sulfate and evaporated to give a white solid. Purification by automated gradient elution flash chromatography (eluent hexane to ethyl acetate) provided the title compound as a white solid, 98 mg, 0.203 mmol, 79%.

### N-[8-Bromo-3-(4-chloro-phenyl)-2-isopropyl-4-oxo-3,4-dihydro-quinazolin-7-yl]-acrylamide

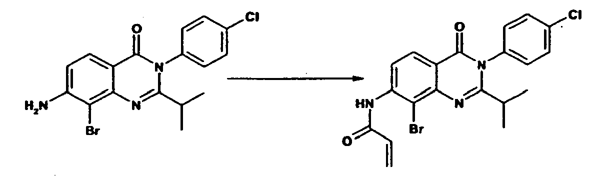

To a solution of 7-Amino-8-bromo-3-(4-chloro-phenyl)-2-isopropyl-3H-quinazolin-4-one (60 mg, 0.153 mmol) in dichloromethane in a microwave vial was added triethylamine (0.032 ml, 0.229 mmol) followed by acyryloyl chloride (0.014 ml, 0.168 mmol). The vial was sealed with a crimp cap and the mixture was heated to 150 °C for 3000 s under microwave irradiation. TLC and LCMS analysis indicated complete conversion. The reaction mixture was evaporated and the residue was divided between water and dichloromethane. The organic phase was dried over anhydrous sodium sulfate and evaporated to give a yellow oil. Purification by automated gradient elution flash chromatography (eluent hexane to hexane/ethyl acetate 7/3) provided the title compound as a yellow solid, 57 mg, 0.127 mmol, 84%.

### N-[3-(4-Chlorophenyl)-2-isopropyl-4-oxo-8-vinyl-3,4-dihydro-quinazolin-7-yl]-acetamide

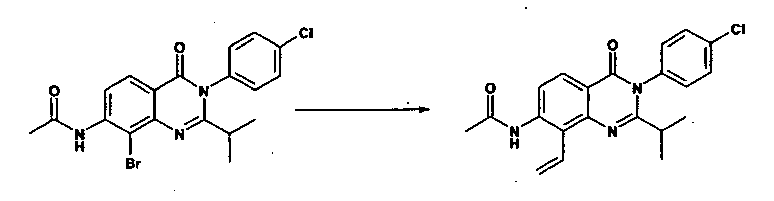

To a suspension of N-[8-Bromo-3-(4-chloro-phenyl)-2-isopropyl-4-oxo-3,4-dihydro-quinazolin-7-yl]-acetamide (250 mg, 0.573 mmol) in toluene/THF 2/1 (6 ml) in a microwave vial was added tributyl(vinyl)tin (0.184 ml, 0.629 mmol) and (tetrakistriphenylphosphine)palladium(0) (66 mg, 0.057 mmol). The vial was sealed with a crimp cap and the mixture was heated to 120 °C for 1 h under microwave irradiation. TLC analysis indicated starting material remained. THF (1 ml) was added to the vial, which was re-sealed and heated to 150 °C for 1 h under microwave irradiation. TLC showed completion of the reaction. The reaction mixture was filtered through Celite, washing with methanol. The solution was evaoporated and the residue was purified by automated gradient elution flash chromatography (silica 12 g, eluent hexane (+2% triethylamine) to hexane (+2% triethylamine)/ethyl acetate 1/1) to provide the title compound as a white solid, 159 mg, 0.414 mmol, 72%.

### N-Allyl-N-[3-(4-chlorophenyl)-2-isopropyl-4-oxo-8-vinyl-3,4-dihydroquinazolin-7-yl]-acetamide

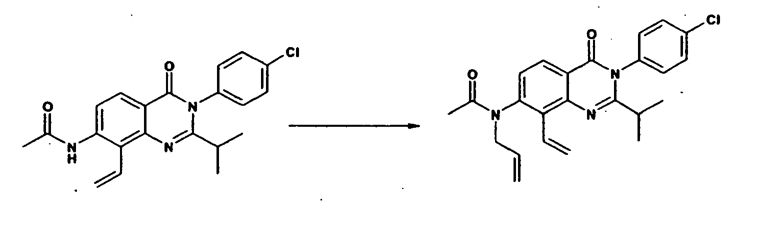

To a solution of of N-[3-(4-Chlorophenyl)-2-isopropyl-4-oxo-8-vinyl-3,4-dihydroquinazolin-7-yl]-acetamide (155 mg, 0.404 mmol) in DMF (3 ml) was added allyl bromide (0.037 ml, 0.42 mmol) and cesium carbonate (270 mg, 0.82 mmol). The suspension was stirred for 1 h at ambient temperature then heated to 60 °C (external temperature) in an oil bath. TLC showed completion of the reaction. The mixture was diluted with water and ethyl acetate and the aqueous phase was extracted with ethyl acetate (x 2). The combined organic phases were dried over anhydrous sodium sulfate and evaporated. The residue was purified by automated gradient elution flash chromatography (silica 12 g, eluent hexane to hexane/ethyl acetate 1/1) to provide the title compound as a white foam, 156 mg, 0.37 mmol, 91 %.

### N-[3-(4-Chloro-phenyl)-2-isopropyl-4-oxo-8-vinyl-3,4-dihydro-quinazolin-7-yl]-N-(2-methyl-allyl)-acetamide

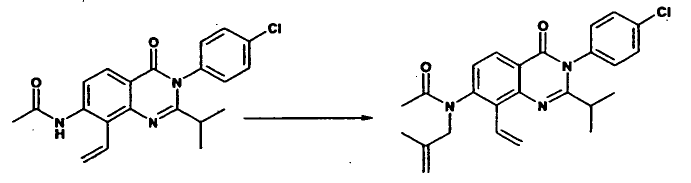

The title compound was prepared in an analogous manner to N-Allyl-N-[3-(4-chlorophenyl)-2-isopropyl-4-oxo-8-vinyl-3,4-dihydroquinazolin-7-yl] acetamide using 3-bromo-2-methylpropene instead of allyl bromide. Yield 89%.

### N-[3-(4-Chloro-phenyl)-8-iodo-2-isopropyl-4-oxo-3,4-dihydro-quinazolin-7-yl]-N-(4methylpent-3-enyl)acetamide

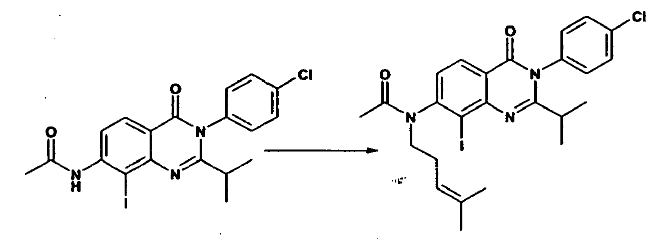

To a solution of N-[3-(4-Chloro-phenyl)-8-iodo-2-isopropyl-4-oxo-3,4-dihydro-quinazolin-7-yl]-acetamide (150 mg, 0.312 mmol) in DMF (2 ml) in a microwave vial was added cesium carbonate (203 mg, 0.624 mmol) and 5-bromo-2-methyl-2-pentene (0.046 ml, 0.343 mmol). The vial was sealed with a crimp cap and the mixture was heated to 80 °C for 20 min under microwave irradiation. Additional 5-bromo-2-methyl-2-pentene (0.046 ml, 0.343 mmol) was added and the mixture was heated to 80 °C for 1 h under microwave irradiation. TLC and LCMS analysis indicated complete conversion. The reaction mixture was diluted with water, extracted with ethyl acetate (x 3) and the combined organic extracts were dried over anhydrous sodium sulfate, and evaporated. The residue was purified by automated gradient elution flash chromatography (eluent hexane to 15% ethyl acetate in hexane) to provide the title compound as a colourless oil (136 mg, 0.241 mmol, 77%).

### N-But-3-enyl-N-[3-(4-chloro-phenyl)-8-iodo-2-isopropyl-4-oxo-3,4-dihydro-quinazolin-7-yl]-acetamide

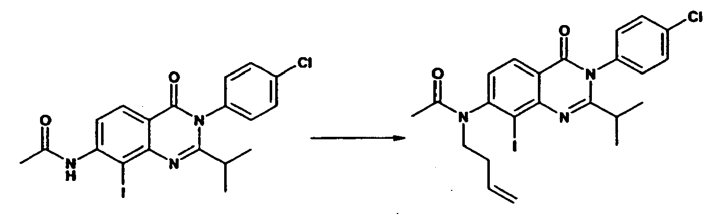

The title compound was prepared in an analogous manner to **N-[3-(4-Chlorophenyl)-8-iodo-2-isopropyl-4-oxo-3,4-dihydro-quinazolin-7-yl]-N-(4methylpent-3-enyl)acetamide** using 4-bromobutene instead of 5-bromo-2-methyl-2-pentene. Purification by automated gradient elution flash chromatography (eluent hexane to 15% ethyl acetate in hexane) provided the title compound as a clear oil, 61%.

### N-[8-Allyl-3-(4-chlorophenyl)-2-isopropyl-4-oxo-3,4-dihydro-quinazolin-7-yl]-acetamide

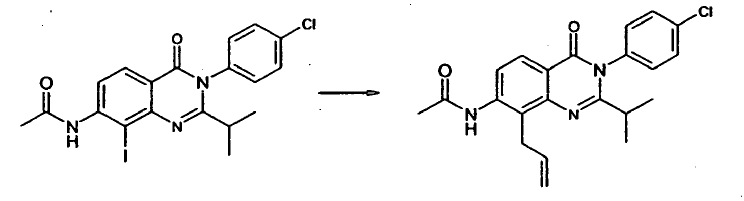

To a solution of N-[3-(4-Chloro-phenyl)-8-iodo-2-isopropyl-4-oxo-3,4-dihydro-quinazolin-7-yl]-acetamide (0.2g, 0.447mmole) and Pd(PPh₃)₄ (0.077g, 0.067mmole) in toluene(5ml) was added allyltributyltin (0.17ml, 0.5mmole). The resulting mixture was heated in the microwave for 1hr at 140°C. The mixture was cooled down to room temperature, filtered through celite and the pad washed with EtOAc. The filtrate was washed with HCl (2M), the organic phase was dried over MgSO4 (anhydrous) and concentrated. The crude material was purified by flash chromatography(10% EtOAc/cyclohexane) to give 0.1g (62%) of N-(8-Allyl-3-(4-chlorophenyl)-2-isopropyl-4-oxo-3-phenyl-3,4-dihydro-quinazolin-7-yl)-acetamide.

### 7-Amino-3-(4-chloro-phenyl)-2-isopropyl-8-trimethylsilanylethynyl-3H-quinazolin-4-one

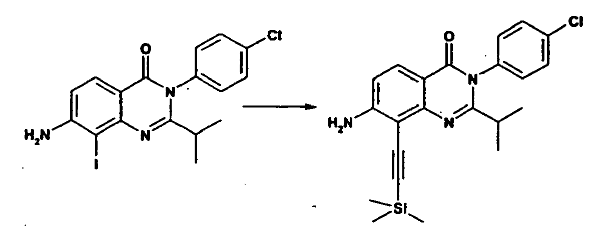

A mixture of 7-Amino-3-(4-chloro-phenyl)-8-iodo-2-isopropyl-3H-quinazolin-4-one (100 mg, 0.229 mmol), (tetrakistriphenylphosphine)palladium(0) (52 mg, 0.044 mmol), trimethylsilylacetylene (0.162 ml, 1.14 mmol), triethylamine (0.158 ml, 1.14 mmol), copper(I) iodide (18.4 mg, 0.088 mmol) and DMF was sealed in a microwave vial with a crimp cap. The reaction mixture was heated to 60 °C for 1.5 h under microwave irradiation. The mixture was evaporated and the residue was purified by automated gradient elution flash chromatography (eluent hexane to ethyl acetate) to provide the title compound (55 mg, 0.134 mmol, 59%).

### 7-Amino-3-(4-chloro-phenyl)-8-ethynyl-2-isopropyl-3H-quinazolin-4-one

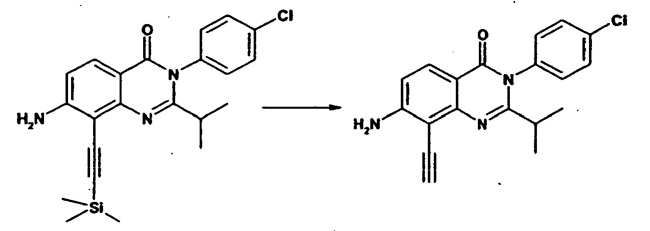

To a solution of 7-Amino-3-(4-chloro-phenyl)-2-isopropyl-8-trimethylsilanylethynyl-3H-quinazolin-4-one (90 mg, 0.22 mmol) in THF (8 ml) was added tetrabutylammonium fluoride (1 M in THF, 0.247 ml, 0.247 mmol). The reaction mixture was stirred at ambient temperature overnight. The reaction mixture was partitioned between water/ethyl acetate and the aqueous layer was extracted with ethyl acetate. Evaporation gave the title compound (74 mg, 0.219 mmol, 100%), which was used without further purification.

### EXAMPLE 1

### 7-(4-Chloro-phenyl)-6-isopropyl-2,3-dihydro-1H,7H-4-oxa-1,5,7-triazaphenanthren-8-one

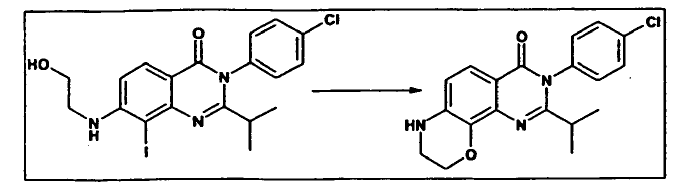

To an oven-dried microwave vial was added 3-(4-Chloro-phenyl)-7-(2-hydroxyethylamino)-8-iodo-2-isopropyl-3H-quinazolin-4-one (70 mg, 0.145 mmol), cesium carbonate (94 mg, 0.290 mmol), copper(I) iodide (27.6 mg, 0.145 mmol) and acetonitrile. The vial was sealed with a crimp cap and the mixture was heated to 150 °C for 5000 s under microwave irradiation. TLC and LCMS analysis indicated complete conversion. The reaction mixture was partitioned between water and ethylacetate. The organic phase was washed with water, dried over anhydrous sodium sulfate and evaporated to give a dark oil. Purification by automated preparative hplc provided the title compound as a white solid, 14 mg, 0.039 mmol, 27%. %). ¹H nmr δ_{H} (400 MHz, CDCl₃) 7.69 (1 H, d), 7.50 (2 H, d), 7.19 (2 H, d), 6.69 (1 H, d), 4.45 (2 H, t), 4.27 (1 H, brs), 3.57 (2 H, t), 2.67 (1 H, m), 1.23 (6 H, d).

### EXAMPLE 2

### 3-(4-Chloro-phenyl)-2-isopropyl-9-methyl-7,9-dihydro-3H-pyrrolo[2,3-h]quinazoline-4,8-dione

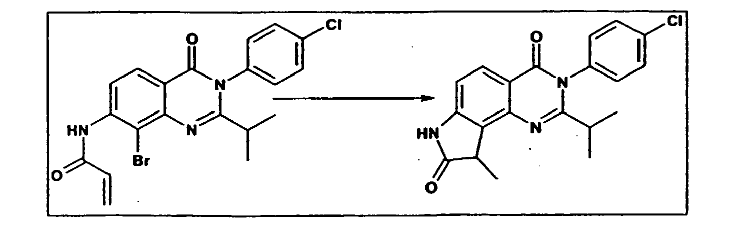

To a solution of N-[8-Bromo-3-(4-chloro-phenyl)-2-isopropyl-4-oxo-3,4-dihydro-quinazolin-7-yl]-acrylamide (41 mg, 0.092 mmol) in acetonitrile (4.5 ml) in a microwave vial was added (tetrakistriphenylphosphine)palladium(0) (10 mg, 0.0086 mmol), followed by triethylamine (0.064 ml, 0.46 mmol). The vial was sealed with a crimp cap and the mixture was heated to 150 °C for 5000 s under microwave irradiation. TLC analysis indicated complete conversion. The reaction mixture was filtered through Celite. The filtrate was evaoporated and the yellow residue was purified by automated gradient elution flash chromatography (silica 12 g, eluent hexane to hexane/ethyl acetate 7/3) to provide the title compound as a yellow solid, 10.5 mg, 0.028 mmol, 31%). ¹H nmr δ_{H} (400 MHz, CDCl₃) 8.18 (1 H, d), 7.53 (2 H, d), 7.21 (2 H, d), 7.01 (1 H, d), 3.85 (1 H, q), 2.65 (1 H, m), 1.76 (3 H, d), 1.23 (6 H, m).

### EXAMPLE 3

### 3-(4-Chloro-phenyl)-2-cyclopropyl-9-methyl-7,9-dihydro-3H-pyrrolo[2,3-h]quinazoline-4,8-dione

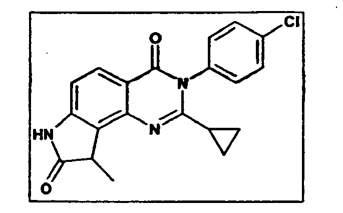

Prepared analogously to Example 2.
MH+ 366. HPLC ret time 4.6 min. 1H NMR (400 MHz, CDCl3): 8.18 (d, 1H), 8.16 (s,1H), 7.5 (d, 2H), 7.3 (d, 2H), 6.9 (d, J= 8.3Hz, 1H), 1.7 (d, 3H), 1.4 (t, 2H), 1.3 (m, 1H), 1.2 (m,1H), 0.9 (t, 2H).

### EXAMPLE 4

### 7-Acetyl-3-(4-chloro-phenyl)-2-isopropyl-7,8-dihydro-3H-pyrido[2,3-h]quinazolin-4-one

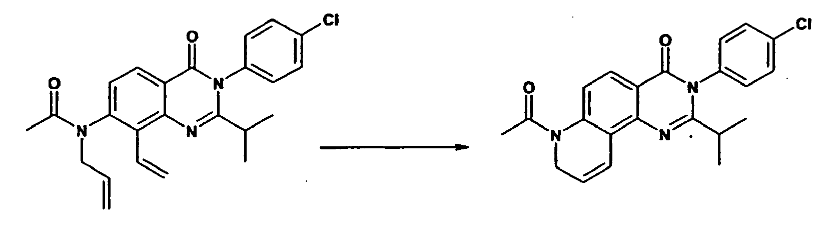

To a solution of N-Allyl-N-[3-(4-chlorophenyl)-2-isopropyl-4-oxo-8-vinyl-3,4-dihydroquinazolin-7-yl]-acetamide (62 mg, 0.147 mmol) in DCM (5 ml) in a microwave vial was added Grubbs second generation ring closing metathesis catalyst (10.6 mg, 0.012 mmol). The vial was sealed with a crimp cap and the mixture was heated to 60 °C for 1 h under microwave irradiation. TLC showed completion of the reaction. The mixture was evaporated and the residue was purified by automated gradient elution flash chromatography (silica 4 g, eluent hexane to hexane/ethyl acetate 3/2) to provide the title compound as a colourless foam, 55.5 mg, 0.141 mmol, 96%.

### EXAMPLE 5

### 7-Acetyl-3-(4-chloro-phenyl)-2-isopropyl-9-methyl-7,8-dihydro-3H-pyrido[2,3-h]quinazolin-4-one

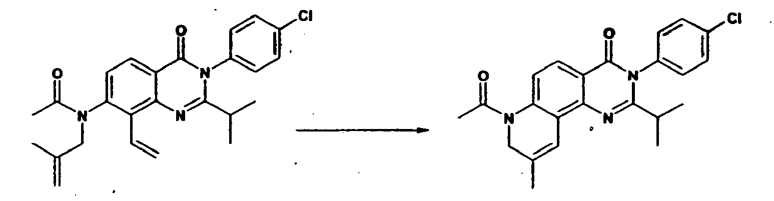

The title compound was prepared in an analogous way to 7-acetyl-3-(4-chlorophenyl)-2-isopropyl-7,8-dihydro-3H-pyrido[2,3-h]quinazolin-4-one using N-[3-(4-chloro-phenyl)-2-isopropyl-4-oxo-8-vinyl-3,4-dihydro-quinazolin-7-yl]-N-(2-methylallyl) acetamide instead of N-allyl-N-[3-(4-chlorophenyl)-2-isopropyl-4-oxo-8-vinyl-3,4-dihydroquinazolin-7-yl] acetamide, increasing the catalytic loading to 20 mol% and extending the reaction time to 17 h. Yield 42%.

### EXAMPLE 6

### 7-Acetyl-3-(4-chloro-phenyl)-2-isopropyl-7,8,9,10-tetrahydro-3H-pyrido[2,3-h]quinazolin-4-one

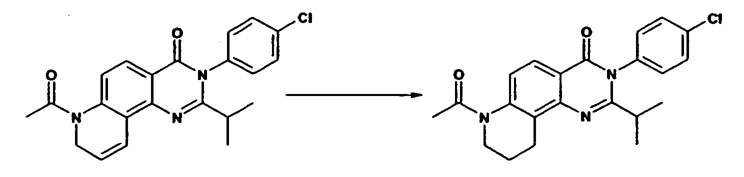

To a solution of 7-Acetyl-3-(4-chlorophenyl)-2-isopropyl-7,8-dihydro-3H-pyrido[2,3-h]quinazolin-4-one (55 mg, 0.139 mmol) in glacial acetic acid (4 ml) was added a catalytic amount of palladium on activated charcoal (10% Pd-C) and the mixture was stirred under an atmosphere of hydrogen (balloon) for 2 h. LCMS analysis showed completion of the reaction. The suspension was filtered through Celite and evaporated to yield the title compound as a pale yellow oil (58.1 mg, >100%), which was used without purification.

### EXAMPLE 7

### 7-Acetyl-3-(4-chloro-phenyl)-2-isopropyl-9-methyl-7,8,9,10-tetrahydro-3H-pyrido[2,3-h]quinazolin-4-one

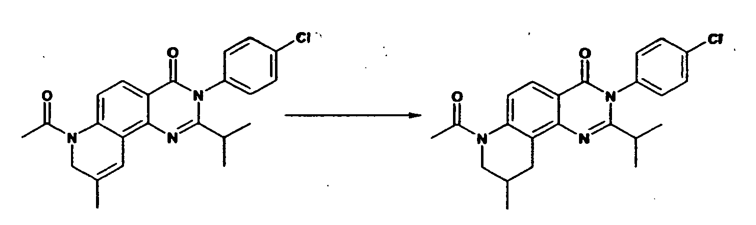

The title compound was prepared in an analogous way to 7-Acetyl-3-(4-chlorophenyl)-2-isopropyl-7,8,9,10-tetrahydro-3H-pyrido[2,3-h]quinazolin-4-one replacing 7-acetyl-3-(4-chlorophenyl)-2-isopropyl-7,8-dihydro-3H-pyrido[2,3-h]quinazolin-4-one with 7-Acetyl-3-(4-chloro-phenyl)-2-isopropyl-9-methyl-7,8-dihydro-3H-pyrido[2,3-h]quinazolin-4-one. Yield 89%.

### EXAMPLE 8

### 3-(4-Chloro-phenyl)-2-isopropyl-7,8,9,10-tetrahydro-3H-pyrido[2,3h]quinazolin-4-one

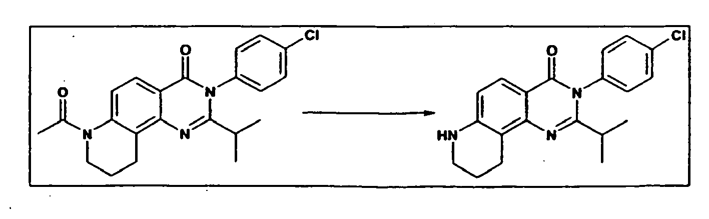

To a solution of 7-Acetyl-3-(4-chloro-phenyl)-2-isopropyl-7,8,9,10-tetrahydro-3H-pyrido[2,3-h]quinazolin-4-one (29 mg, 0.073 mmol) in methanol (2 ml) in a microwave vial was added 2 M HCl (1 ml). The vial was sealed with a crimp cap and the mixture was heated to 100 °C for 0.5 h under microwave irradiation. TLC showed completion of the reaction. The reaction mixture was basified to pH 9 by addition of saturated aqueous sodium hydrogen carbonate and extracted with ethyl acetate (x 3). The combined organic layers were dried over anhydrous sodium sulfate and evaporated. The residue was purified by automated gradient elution flash chromatography (silica 4 g, eluent hexane to hexane/ethyl acetate 3/2) to provide the title compound as a white crystalline solid, 11.9 mg, 0.034 mmol, 46%. ¹H nmr δ_{H} (400 MHz, CDCl₃) 7.86 (1 H, d), 7.49 (2 H, d), 7.17 (2 H, d), 6.55 (1 H, d), 4.40 (1 H, brs), 3.40 (2 H, t), 3.12 (2 H, t), 2.61 (1 H, m), 2.00 (2 H, m), 1.19 (6 H, d).

### EXAMPLE 9

### 3-(4-Chloro-phenyl)-2-isopropyl-9-methyl-7,8,9,10-tetrahydro-3H-pyrido [2,3-h]quinazolin-4-one

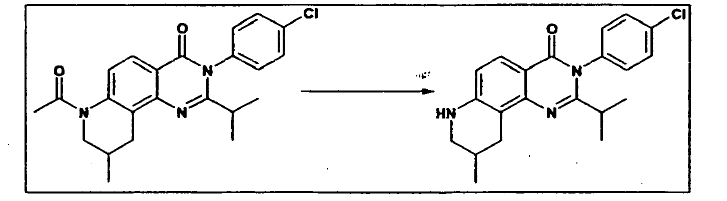

The title compound was prepared in an analogous way to 3-(4-Chloro-phenyl)-2-isopropyl-7,8,9,10-tetrahydro-3H-pyrido[2,3h]quinazolin-4-one replacing 7-acetyl-3-(4-chloro-phenyl)-2-isopropyl-7,8,9,10-tetrahydro-3H-pyrido[2,3-h]quinazolin-4-one with 7-acetyl-3-(4-chloro-phenyl)-2-isopropyl-9-methyl-7,8,9,10-tetrahydro-3H-pyrido[2,3-h]quinazolin-4-one. Yield 50%. ¹H nmr δ_{H} (400 MHz, CDCl₃) 7.86 (1 H, d), 7.48 (2 H, d), 7.17 (2 H, d), 6.56 (1 H, d), 4.42 (1 H, brs), 3.44 (1 H, dd), 3.37 (1 H, brd), 2.98 (1 H, t), 2.61 (1 H, m), 2.48 (1 H, dd), 2.08 (brm), 1.20 (3 H, d), 1.14 (3 H, d).

### EXAMPLE 10

### 7-Acetyl-3-(4-chloro-phenyl)-2-isopropyl-9-methyl-3H,7H-pyrrolo[2,3-h]quinazolin-4-one, 3-(4-Chloro-phenyl)-2-isopropyl-9-methyl-3H,7H-pyrrolo[2,3-h]quinazolin-4-one and 7-Acetyl-3-(4-chloro-phenyl)-2-isopropyl-9-methylene-8,9-dihydro-3H,7H-pyrrolo[2,3-h]quinazolin-4-one

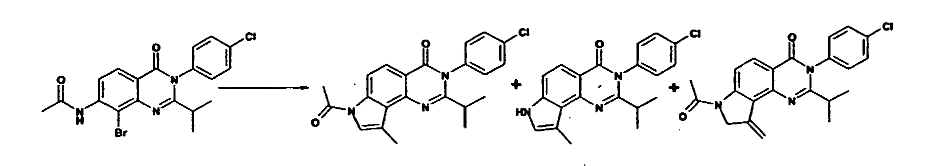

To a solution of N-[8-Bromo-3-(4-chloro-phenyl)-2-isopropyl-4-oxo-3,4-dihydro-quinazolin-7-yl]-acetamide (92 mg, 0.211 mmol) in DMF ( 3 ml) in a microwave vial was added cesium carbonate (140 mg, 0.43 mmol) and allyl bromide (0.02 ml, 0.231 mmol). The reaction mixture was stirred at ambient temperature overnight. TLC and LCMS analysis indicated complete loss of starting material. (Tetrakistriphenylphosphine)palladium(0) (40 mg, 0.035 mmol) was added, the vial was re-sealed with a crimp cap and the mixture was heated to 100 °C for 20 min under microwave irradiation. LCMS analysis indicated complete loss of the N-allyl intermediate. The reaction mixture was evaporated and the residue was divided between ethyl acetate/water. The aqueous layer was extracted with ethyl acetate (x 2) and the combined organic extracts were dried over anhydrous sodium sulfate and evaporated. The residue was purified by automated gradient elution flash chromatography (silica 12 g, eluent hexane to hexane/ethyl acetate 1/1) to provide the title compounds: **7-Acetyl-3-(4-chloro-phenyl)-2-isopropyl-9-methyl-3H,7H-pyrrolo[2,3-h]quinazolin-4-one** (eluting first, 8.8 mg, 0.022 mmol, 11%); **3-(4-Chloro-phenyl)-2-isopropyl-9-methyl-3H,7H-pyrrolo[2,3-h]quinazolin-4-one** (eluting second, 6 mg, 0.015 mmol, 8%) and **7-Acetyl-3-(4-chloro-phenyl)-2-isopropyl-9-methylene-8,9-dihydro-3H,7H-pyrrolo[2,3h]quinazolin-4-one** (eluting third, 48 mg, 0.122 mmol, 58%). Total recovery: 77%.

### EXAMPLE 11

### 7-Acetyl-3-(4-chloro-phenyl)-2-isopropyl-9-methyl-3H,7H-pyrrolo[2,3h]quinazolin-4-one

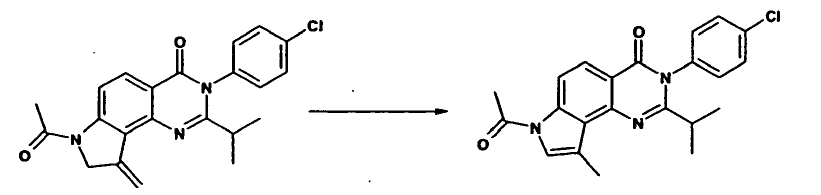

To a solution of **7-Acetyl-3-(4-chloro-phenyl)-2-isopropyl-9-methylene-8,9-dihydro-3H,7H-pyrrolo[2,3h]quinazolin-4-one** (48 mg, 0.122 mmol) in DCM (2 ml) was added camphorsulfonic acid (29 mg, 0.122 mmol). The reaction mixture was stirred for 20 h at room temperature. LCMS showed complete loss of starting material. The reaction mixture was diluted with water and ethyl acetate. The aqueous layer was extracted with ethyl acetate (x 2) and the combined organic extracts were dried over anhydrous sodium sulfate, and evaporated to give the title compound (48 mg, 0.122 mmol, 100%), which was used without purification.

### EXAMPLE 12

### 3-(4-Chloro-phenyl)-2-isopropyl-9-methyl-3H,7H-pyrrolo[2,3-h]quinazolin-4-one

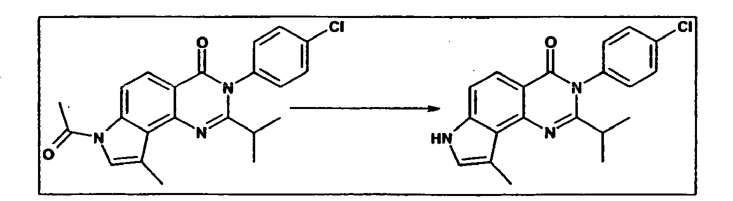

To a solution of of 7-Acetyl-3-(4-chloro-phenyl)-2-isopropyl-9-methylene-8,9-dihydro-3H,7H-pyrrolo[2,3h]quinazolin-4-one (48 mg, 0.122 mmol) in methanol/water 2/1 (3 ml) in a microwave vial was added potassium carbonate (150 mg, 1.087 mmol). The vial was sealed with a crimp cap and the mixture was heated to 120 °C for 0.5 h under microwave irradiation. TLC analysis showed completion of the reaction. The reaction mixture was diluted with water and ethyl acetate. The aqueous layer was extracted with ethyl acetate (x 2) and the combined organic extracts were dried over anhydrous sodium sulfate, and evaporated. The residue was purified by automated gradient elution flash chromatography (silica 4 g, eluent hexane to hexane/ethyl acetate 1/1) to provide the title compound as a yellow solid (25.4 mg, 0.072 mmol, 59%). ¹H nmr δ_{H} (400 MHz, CDCl₃) 8.33 (1 H, brs), 8.00 (1 H, d), 7.53 (2 H, d), 7.39 (1 H, d), 7.23 (2 H, d), 7.06 (1 H, m), 2.76 (3 H, s) 2.71 (1 H, m), 1.27 (6 H, d).

### EXAMPLE 13

### 7-Acetyl-3-(4-chloro-phenyl)-10-isopropenyl-2-isopropyl-7,8,9,10-tetrahydro-3H-pyrido[2,3-h]quinazolin-4-one

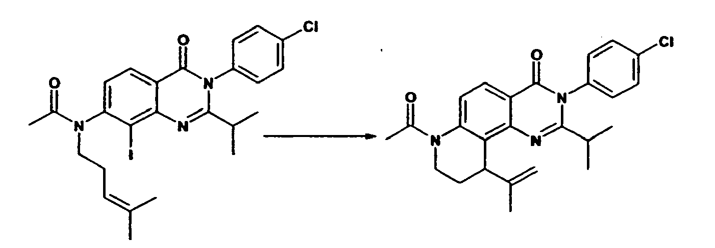

To an oven-dried, microwave vial was added N-[3-(4-Chtoro-phenyl)-8-iodo-2-isopropyl-4-oxo-3,4-dihydro-quinazolin-7-yl]-N-(4methylpent-3-enyl)acetamide (61 mg, 0.108 mmol), (tetrakistriphenylphosphine)palladium(0) (25 mg, 0.022mmol), cesium carbonate (176 mg, 0.541 mmol) and DMF (2.5 ml). The vial flushed with nitrogen, sealed with a crimp cap and the mixture was heated to 120 °C for 1 h under microwave irradiation. TLC and LCMS analysis indicated completion. The reaction mixture was poured into water, extracted with ethyl acetate (x 2) and the combined organic extracts were dried over anhydrous sodium sulfate, and evaporated. The residue was purified by automated gradient elution flash chromatography (eluent hexane to 15% ethyl acetate in hexane) to provide the title compound as a clear, pale yellow oil (46 mg, 0.105 mmol, 98%).

### EXAMPLE 14

### 7-Acetyl-3-(4-chloro-phenyl)-2,10-diisopropyl-7,8,9,10-tetrahydro-3H-pyrido[2,3-h]quinazolin-4-one

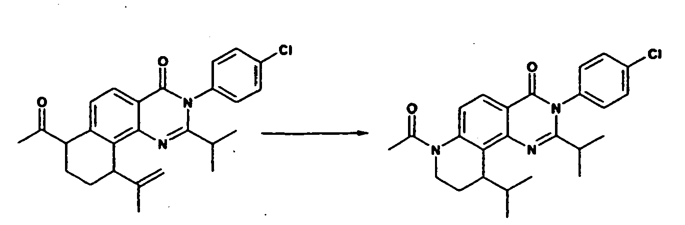

To a solution of 7-Acetyl-3-(4-chtoro-phenyl)-10-isopropenyl-2-isopropyl-7,8,9,10-tetrahydro-3H-pyrido[2,3-h]quinazolin-4-one (44 mg, 0.101 mmol) in absolute ethanol/glacial acetic acid 1/1 (3 ml) was added a catalytic amount of palladium on activated charcoal (10% Pd-C, 10 mg) and the mixture was stirred under an atmosphere of hydrogen (balloon) for 5 h. LCMS analysis showed completion of the reaction. The suspension was filtered through Celite and the filtrate was neutralized with saturated aqueous sodium hydrogen carbonate. The aqueous solution was extracted with dichloromethane and the organic extracts were evaporated. The residue was purified by automated gradient elution flash chromatography (eluent hexane to 20% ethyl acetate in hexane) to provide the title compound as a clear oil (37 mg, 0.084 mmol, 84%).

### EXAMPLE 15

### 3-(4-Chloro-phenyl)-2,10-diisopropyl-7,8,9,10-tetrahydro-3H-pyrido[2,3-h]quinazolin-4-one

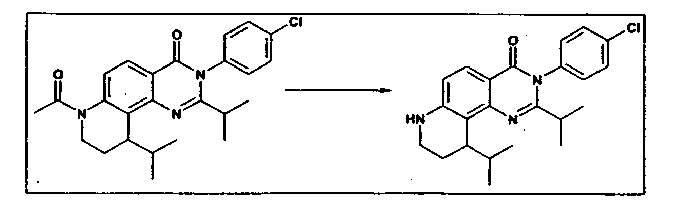

To a solution 7-Acetyl-3-(4-chloro-phenyl)-2,10-diisopropyl-7,8,9,10-tetrahydro-3H-pyrido[2,3-h]quinazolin-4-one (30 mg, 0.068 mmol) in methanol (1 ml) in a microwave vial was added aqueous potassium hydroxide solution (1 M, 1 ml). The vial was sealed with a crimp cap and the mixture was heated to 100 °C for 10 min under microwave irradiation. TLC and LCMS analysis indicated completion. The reaction mixture was diluted with dilute hydrochloric acid and extracted with ethyl acetate (x 2). The combined organic extracts were washed with saturated aqueous sodium hydrogen carbonate, dried over anhydrous sodium sulfate and evaporated to give a clear oil (24 mg, 0.060 mmol, 89%). ¹H nmr δ_{H} (400 MHz, CDCl₃) 7.88 (1 H, d), 7.48 (2 H, m), 7.22 (1 H, m), 7.16 (1 H, m), 6.55 (1 H, d), 4.45 (1 H, brs), 3.61 (1 H, m), 3.44 (2 H, m), 2.62 (1 H, m), 2.14 (1 H, dd), 1.93 (1H, m), 1.69 (1 H, m), 1.19 (6 H, m), 1.04 (3 H, d), 0.90 (3 H, d).

### EXAMPLE 16

### 7-Acetyl-3-(4-chlorophenyl)-2-isopropyl-10-methylene-7,8,9,10-tetrahydro-3H-pyrido[2,3-h]quinazolin-4-one and 7-Acetyl-3-(4-chlorophenyl)-2-isopropyl-10-methyl-7,8-dihydro-3H-pyrido[2,3-h]quinazolin-4-one

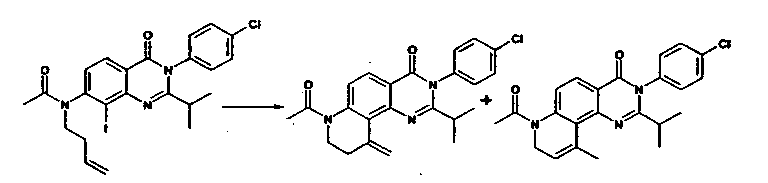

To an oven-dried, microwave vial was added but-3-enyl-N-[3-(4-chloro-phenyl)-8-iodo-2-isopropyl-4-oxo-3,4-dihydro-quinazolin-7-yl]-acetamide (350 mg, 0.654 mmol), DMF (5 ml). (tetrakistriphenylphosphine)palladium(0) (151 mg, 0.131 mmol) and cesium carbonate (1.1 g, 3.4 mmol). The vial was flushed with nitrogen, sealed with a crimp cap and the mixture was heated to 100 °C for 1 h under microwave irradiation. TLC and LCMS analysis indicated completion. The reaction mixture was filtered through Celite and evaporated. Ethyl acetate/water was added to the residue and the organic layer was washed with water, dried over anhydrous sodium sulfate and evaporated to give a dark oil. The residue was purified by automated gradient elution flash chromatography (eluent hexane to 25% ethyl acetate in hexane) to provide the title isomeric compounds: **7-Acetyl-3-(4-chlorophenyl)-2-isopropyl-10-methylene-7,8,9,10-tetrahydro-3H-pyrido[2,3-h]quinazolin-4-one** (108 mg, 0.265 mmol, 41%); and **7-Acetyl-3-(4-chlorophenyl)-2-isopropyl-10-methyl-7,8-dihydro-3H-pyrido[2,3-h]quinazolin-4-one** (138 mg, 0.338 mmol, 52%). Total recovery: 93%.

### EXAMPLE 17

### 7-Acetyl-3-(4-chloro-phenyl)-2-isopropyl-10-methyl-7,8,9,10-tetrahydro-3H-pyrido[2,3-h]quinazolin-4-one

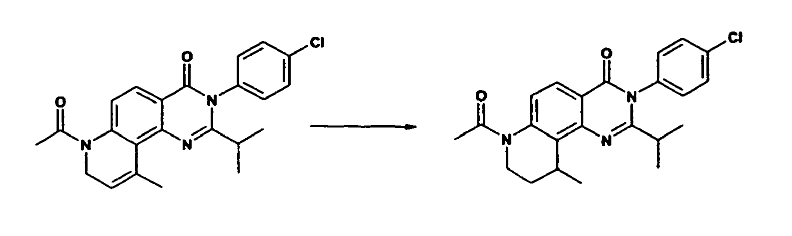

To a solution of 7-Acetyl-3-(4-chlorophenyl)-2-isopropyl-10-methyl-7,8-dihydro-3H-pyrido[2,3-h]quinazolin-4-one (100 mg, 0.246 mmol) in absolute ethanol/glacial acetic acid 1/10 (5.5 ml) was added a catalytic amount of palladium on activated charcoal (10% Pd-C, 10 mg) and the mixture was stirred under an atmosphere of hydrogen (balloon) for 20 h. LCMS analysis showed completion of the reaction. The suspension was filtered through Celite and evaporated. The residue was purified by automated gradient elution flash chromatography (eluent hexane to 30% ethyl acetate in hexane) to provide the title compound as a clear oil (89 mg, 0.218 mmol, 89%).

### EXAMPLE 18

### 3-(4-Chloro-phenyl)-2-isopropyl-10-methyl-7,8,9,10-tetrahydro-3H-pyrido[2,3-h]quinazolin-4-one

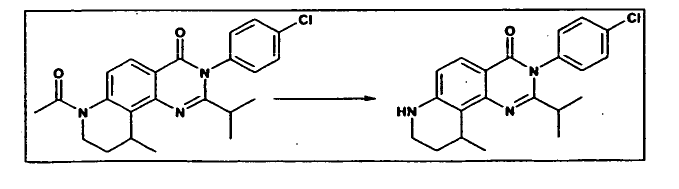

To a solution of 7-Acetyl-3-(4-chloro-phenyl)-2-isopropyl-10-methyl-7,8,9,10-tetrahydro-3H-pyrido[2,3-h]quinazolin-4-one (84.5 mg, 0.207 mmol) in methanol (5 ml) in a microwave vial was added aqueous potassium hydroxide solution (5 M, 1 ml). The vial was sealed with a crimp cap and the mixture was heated to 100 °C for 10 min under microwave irradiation. TLC and LCMS analysis indicated completion. The reaction mixture was diluted with water and extracted with ethyl acetate (x 2). The combined organic extracts were dried over anhydrous sodium sulfate and evaporated. The residue was purified by automated gradient elution flash chromatography (eluent hexane to 20% ethyl acetate in hexane) to provide the title compound as a white solid (60 mg, 0.163 mmol, 79%). %). ¹H nmr δ_{H} (400 MHz, CDCl₃) 7.86 (1 H, d), 7.48 (2 H, d), 7.18 (2 H, m), 6.54 (1 H, d), 4.49 (1 H, brs), 3.83 (1 H, m), 3.48 (1 H, m), 3.36 (1 H, m), 2.61 (1 H, m), 1.93 (1 H, m), 1.82 (1 H, m), 1.33 (3 H, d), 1.21 (6 H, m).

### EXAMPLE 19

### 3-(4-Chloro-phenyl)-2-isopropyl-10-methylene-7,8,9,10-tetrahydro-3H-pyrido[2,3-h]quinazolin-4-one

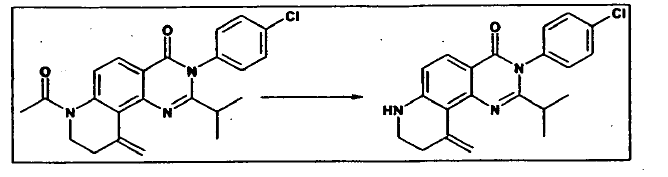

To a solution of 7-Acetyl-3-(4-chlorophenyl)-2-isopropyl-10-methylene-7,8,9,10-tetrahydro-3H-pyrido[2,3-h]quinazolin-4-one (60mg, 0.147 mmol) in methanol (3 ml) in a microwave vial was added aqueous potassium hydroxide solution (5 M, 1 ml). The vial was sealed with a crimp cap and the mixture was heated to 100 °C for 10 min under microwave irradiation. TLC and LCMS analysis indicated completion. The reaction mixture was acidified to pH 6-7 with hydrochloric acid (2M) and extracted with ethyl acetate (x 2). The combined organic extracts were dried over anhydrous sodium sulfate and evaporated. The residue was purified by automated gradient elution flash chromatography (eluent hexane to 20% ethyl acetate in hexane) to provide the title compound as a white solid (35 mg, 0.096 mmol, 65%). %). ¹H nmr δ_{H} (400 MHz, CDCl₃) 7.90 (1 H, d), 7.49 (2 H, d), 7.19 (2 H, d), 6.63 (1 H, d), 6.55 (1 H, d), 5.32 (1 H, brm), 4.63 (1 h, brs), 3.50 (2 H, brt), 2.66 (3 H, overlapping m), 1.20 (6 H, d).

### EXAMPLE 20

### 7-Acetyl-3-(4-chloro-phenyl)-2-isopropyl-8,9-dihydro-3H,7H-pyrido[2,3-h]quinazoline-4,10-dione

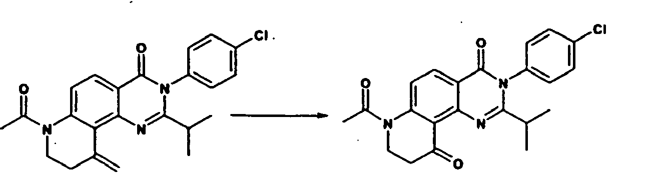

A solution of 7-Acetyl-3-(4-chlorophenyl)-2-isopropyl-10-methylene-7,8,9,10-tetrahydro-3H-pyrido[2,3-h]quinazolin-4-one (85 mg, 0.208 mmol) in methanol/dichloromethane 2/1 (5 ml) was cooled to -78 °C and a steady stream of ozone was bubbled into the reaction mixture over 0.5 h. The reaction mixture was allowed to warm to -20 °C whilst being purged with a steady stream of argon. The solution was then cooled to -78 °C and dimethyl sulfide (3 drops) was added. The stirred solution was allowed to reach ambient temperature overnight and then evaporated. The residue was purified by automated gradient elution flash chromatography (eluent hexane to 80% ethyl acetate in hexane) to provide the title compound as a white solid (38 mg, 0.093 mmol, 44%).

### EXAMPLE 21

### 3-(4-Chloro-phenyl)-2-isopropyl-8,9-dihydro-3H,7H-pyrido[2,3-h]quinazoline-4,10-dione

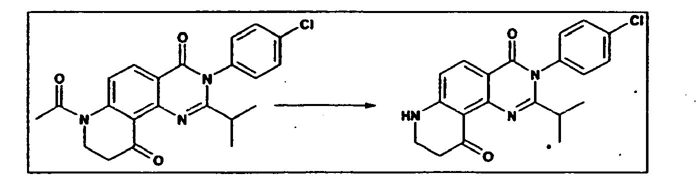

To a solution of 7-Acetyl-3-(4-chloro-phenyl)-2-isopropyl-8,9-dihyoro-3H,7H-pyrido[2,3-h]quinazoline-4,10-dione (30mg, 0.073 mmol) in methanol (4 ml) was added aqueous potassium hydroxide solution (5 M, 1 ml). After 2 min, TLC and LCMS analysis indicated completion. The reaction mixture was diluted with water and extracted with ethyl acetate (x 2). The combined organic extracts were dried over anhydrous sodium sulfate and evaporated. The residue was purified by automated gradient elution flash chromatography (eluent hexane to 70% ethyl acetate) to provide the title compound as a white solid (11.9 mg, 0.032 mmol, 44%). ¹H nmr δ_{H} (400 MHz, CDCl₃) 8.07 (1 H, d), 7.51 (2 H, d), 7.17 (2 H, d), 6.66 (1 H, d), 5.12 (1 H, brs), 3.70 (2 H, brm), 2.81 (2 H, t), 2.66 (1 H, m), 1.26 (6 H, d).

### EXAMPLE 22

### 7-Acetyl-3-(4-chlorophenyl)-2-isopropyl-8-methyl-3H,7H-pyrrolo[2,3-h]quinazolin-4-one

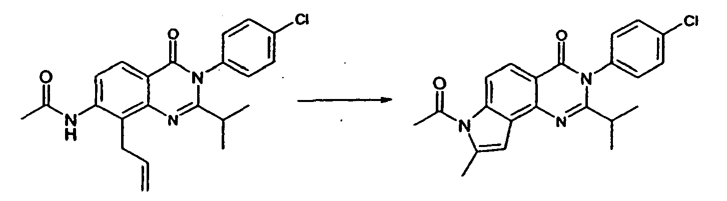

To a solution of N-(8-Allyl-2-isopropyl-4-oxo-3-phenyl-3,4-dihydro-quinazolin-7-yl)-acetamide(2)(0.02g, 0.05mmole) in THF(3ml) was added PdCl2(MeCN)2 (0.013g, 0.05mmole) and stirred at room temperature for 1hr. To this solution was added Et3N(0.02ml, 0.15mmole) and stirred for 2hrs. Reaction mixture was filtered through celite and washed pad with EtOAc. The filtrate was concentrated and purified by flash chromatography(10% EtOAc/cyctohexane) to give 0.01g (51%) of 7-Acetyl-3-(4-chlorophenyl)-2-isopropyl-8-methyl-3H,7H-pyrrolo[2,3-h]quinazolin-4-one.

### EXAMPLE 23

### 3-(4-Chlorophenyl)-2-isopropyl-8-methyl-3H,7H-pyrrolo[2,3-h]quinazolin-4-one

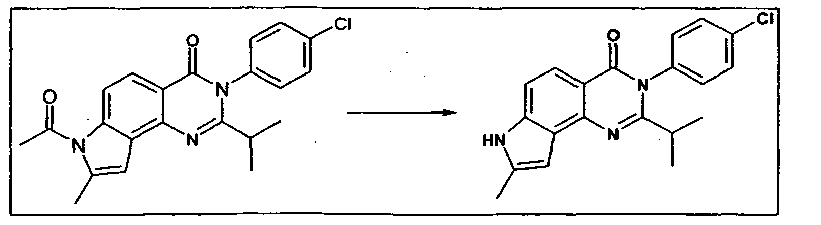

To a solution of 7-Acetyl-3-(4-chloro-phenyl)-2-isopropyl-8-methyl-3H,7H-pyrrolo[2,3-h]quinazolin-4-one(3)(0.02g, 0.05mmole) in MeOH(0.5m) was added 2ml of KOH (5M) and heated in the microwave at 100°C for 2hrs. Reaction mixture was diluted with H2O, extracted with EtOAc,the organic phase was dried over MgSO4 (anhydrous) and concentrated to give 0.016g (90%) of 3-(4-Chlorophenyl)-2-isopropyl-8-methyl-3H,7H-pyrrolo[2,3-h]quinazolin-4-one. MH+ = 352. HPLC retention time = 5.7 min. δH (400MHz, CDCl₃) 8.28 (s, 1H), 7.9 (d,1H), 7.5 (d, 2H), 7.3 (d, 1H), 7.2 (d, 2H), 6.8 (s, 1H), 2.7 (m, 1H), 2.5 (s,3H), 1.2 (d, 6H).

### EXAMPLE 24

### 3-(4-Chloro-phenyl)-2-isopropyl-3H,7H-pyrrolo[2,3-h]quinazolin-4-one

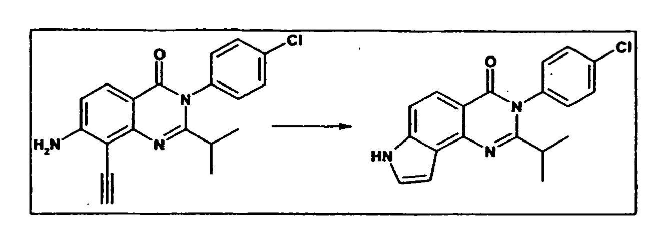

To a suspension of potassium hydride CAUTION PYROPHORIC (30% dispersion in mineral oil, 36 mg, 0.27 mmol) in NMP in an oven-dried, argon-flushed microwave vial was added a solution of 7-Amino-3-(4-chloro-phenyl)-8-ethynyl-2-isopropyl-3H-quinazolin-4-one (33.5 mg, 0.099 mmol) in NMP. The microwave vial was sealed with a crimp cap and the reaction mixture was stirred overnight at room temperature. LCMS analysis indicated 50% completion of the reaction. The reaction mixture was heated at 70 °C for 1 h under microwave irradiation. LCMS analysis indicated completion. The reaction mixture was diluted with water and extracted with ethyl acetate (x 2). The combined organic extracts were dried over anhydrous sodium sulfate and evaporated.
**Alternative procedure:** To a suspension of potassium tertiary-butoxide (36 mg, 0.32 mmol) in NMP in an oven-dried, argon-flushed microwave vial was added a solution of 7-Amino-3-(4-chloro-phenyl)-8-ethynyl-2-isopropyl-3H-quinazolin-4-one (43 mg, 0.127 mmol) in NMP. The microwave vial was sealed with a crimp cap and the reaction mixture was heated at 80 °C for 1 h under microwave irradiation. LCMS analysis indicated completion. The reaction mixture was diluted with water and extracted with ethyl acetate (x 2). The combined organic extracts were dried over anhydrous sodium sulfate and evaporated.
The combined residues for the two procedures was purified by automated gradient elution flash chromatography (eluent 5 to 100% ethyl acetate in hexane) to provide the title compound (40 mg, 0.119 mmol, 52%). ¹H nmr δ_{H} (400 MHz, CDCl₃) 8.54 ( 1 H, brs), 8.04 (1 H, d), 7.52 (2 H, m), 7.47 (1 H, d), 7.33 (1 H, t), 7.23 (3 H, m), 2.72 (1 H, m), 1.26 (6 H, d).

## Claims

1. A quinazolinone compound of the formula 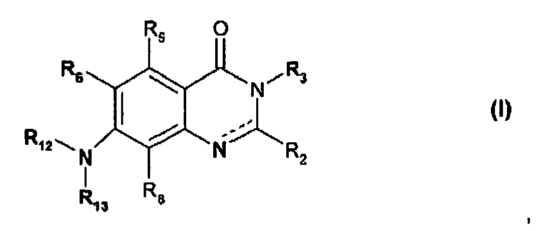 wherein
---- is a single bond or a double bond;
R₂ is selected from
(a) C₁-C₈alkyl, C₃-C₆cycloalkyl, (C₁-C₆alkyl)amino or di-(C₁-C₆alkyl)amino;
or
(b) NH₂, hydroxyC₁-C₆alkylamino-, aminoC₁-C₆alkylamino, C₂-C₆alkenyl, di(trifluoromethyl)C₁-C₆alkyl, R₉-O-(C₁ -C₆alkyl)- in which the alkyl chain is optionally substituted by trifluoromethyl, (NC)-C₁ -C₆alkyl-, (R₁₀R₁₁N-)C₁ - Cealkyl-, (C₁ -C₆alkyl)-SO₂-(C₁ -C₆alkyl)-, wherein R₉, Rio and R₁₁ are each independently H or C₁-C₆ alkyl; phenyl optionally substituted by one, two or three substituents each independently selected from the group consisting of halogen, C₁ -C₆alkyl, halogen-substituted C₁ -C₆alkyl, hydroxy C₁ -C₆alkyl, cyano or a group -(C=O)-R₂ₐ, where R₂ₐ is C₁-C₆alkyl; or 5, 6, or 7-membered, saturated or unsaturated, heterocyclic ring, directly attached to the quinazolinone ring or attached through -C₁ -C₆ alkyl-, containing one, two, or three heteroatoms selected from N, O and S, and optionally substituted with one, two or three substituents selected from C₁ -C₆alkyl, C₁ -C₆alkoxy, hydroxy, cyano, halo, R₁₀R₁₁N-, R₉-O-(C=O)-, -(C=O)-N-R₁₀R₁₁, =O and phenyl ;
R₃ is selected from
(a'):
phenyl substituted by one, two or three substituents each independently selected from the group consisting of halogen, C₁-C₆alkyl, halogen-substituted C₁-C₆alkyl, hydroxyC₁-C₆alkyl, cyano or a group -C(=O)-R₃ₐ, where R₃ₐ is C₁-C₆alkyl; or
(b'):
C₁ -C₆alkyl, (NC)-C₂ -C₆alkyl-, R₉-O-(C₁ -C₆alkyl)-, R₉-O-( C₁ -C₆alkyl)-O-(C₁ - C₆alkyl)-,R₁₀R₁₁N-(C₁ -C₆alkyl)-, R₁₀R₁₁N-(C=O)-(C₁-C₆alkyl)-, or (C₁-C₆alkyl)-SO₂-(C₁ -C₆alkyl)-, wherein R₉, R₁₀ and R₁₁ are each independently H or C₁-C₆ alkyl; or
unsubstituted phenyl, phenyl substituted with one or two substituents selected from -(C₁ -C₆alkoxy)-, R₁₀R₁₁N-, R₁₀R₁₁N-(C₁ -C₆alkyl)-, -SO₂-(C₁-C₆alkyl), R₉-O-(C=O)-, wherein R₉, R₁₀ and R₁₁ are as defined above, or with halo-substituted phenyl or a 5- or 6-membered saturated or unsaturated heterocyclic ring having one, two or three heteroatoms selected from N, O and S and optionally including a further substituent selected from halo, or phenyl substituted with three or four substituents selected from halo, hydroxyl, and C₁ -C₆alkyl; or
a cycloalkyl ring having 3, 4, 5 or 6 carbon atoms, directly attached to the quinazolinone ring or attached through -C₁ -C₆alkyl-, and which is optionally substituted with one or two substituents selected from C₁ -C₆alkyl, C₁ - C₆alkoxy, hydroxy, cyano, halo, R₁₀R₁₁N-, R₉-O-(C=O)-, -(C=O)-N-R₁₀R₁₁, and phenyl; or
benzyl, or phenyl(C₁ -C₆alkyl)-, phenoxy-( C₁ -C₆alkyl)- or phenyl(C=O)-(C₁ - C₆alkyl)-, optionally substituted with one, two, or three substituents selected from C₁ -C₆alkyl, C₁ -C₆alkoxy, hydroxy, cyano, halo, R₁₀R₁₁N-, R₉-O-(C=O)-, -(C=O)-N-R₁₀R₁₁, and phenyl; or
a 5, 6, or 7- membered, saturated or unsaturated, heterocyclic ring, directly attached to the quinazolinone ring or attached through -C₁ -C₆ alkyl-, containing one, two, or three heteroatoms selected from N, O and S, and optionally substituted with one, two or three substituents selected from C₁ - C₆alkyl, C₁ -C₆alkoxy, hydroxy, cyano, halo, R₁₀R₁₁N-, R₉-O-(C=O)-, -(C=O)-N-R₁₀R₁₁, =O and phenyl; or
a 9- or 10- membered aromatic or heterocyclic fused ring, directly attached to the quinazolinone ring or attached through -C₁ -C₆ alkyl-, containing zero, one, two or three heteroatoms selected from N, O and S, and optionally substituted with one, two, three or four substituents selected from C₁ -C₆alkyl, C₁-C₆alkoxy, hydroxy, cyano, halo, R₁₀R₁₁N-, R₉-O-(C=O)-, -(C=O)-N-R₁₀R₁₁, and phenyl; and
R₅ and R₆ are each independently hydrogen, halogen, C₁-C₆alkyl, C₂-C₆alkenyl, C₂-C₆alkynyl, hydroxy, hydroxy-substituted C₁-C₆alkyl, C₁-C₆alkoxy, C₃-C₆cycloalkyl, cyano, -C(=O)H, phenyl, (C₃-C₆cycloalkyl)C₁-C₆alkyl, (C₃-C₆cycloalkyl)C₁-C₆alkoxy, (C₁-C₆alkoxycarbonylamino)C₁-C₆alkoxy or (C₁-C₆alkylcarbonylamino)C₁-C₆alkoxy, (amino) C₁-C₆alkoxy, (dimethylamino)C₁-C₆alkoxy, or (C₁-C₆alkoxycarbonyl) C₁-C₆alkoxy;
R12 is hydrogen, formyl, C₁-C₆alkylcarbonyl or benzyl, the phenyl group of which is optionally substituted by 1, 2 or 3 substituents, selected from the group consisting of halogen, C₁-C₆alkyl, halo-C₁-C₆alkyl, hydroxy, hydroxy-C₁-C₆alkyl, C₁-C₆alkoxy, C₁-C₆alkoxy-C₁-C₆alkyl, halo-C₁-C₆alkoxy, C₁-C₆alkylthio, halo-C₁-C₆alkylthio, C₁-C₆alkylsulfinyl, halo-C₁-C₆alkylsulfinyl, C₁-C₆alkylsulfonyl, halo-C₁-C₆alkylsulfonyl, C₃-C₆cycloalkyl, C₃-C₆cycloalkyl-C₁-C₆alkyl, C₃-C₆cycloalkoxy, C₃-C₆cycloalkoxy-C₁-C₆alkyl, amino, C₁-C₆alkylamino, di-(C₁-C₆alkyl)amino, C₁-C₆alkoxycarbonylamino, cyano, formyl and C₁-C₆alkylcarborlyl, or is substituted at two adjacent carbon atoms by -O-CH₂-O- or -O-CF₂-O-; and
R13 and R8, taken together, represent, together with the three-membered moiety -N-C-C-, to which they are attached, a five-, six-, seven- or eight-membered, partially or fully unsaturated, optionally substituted, heterocyclic ring, which contains 1 ring nitrogen atom and optionally either 1 further ring nitrogen, oxygen or sulfur atom or 2 further ring nitrogen atoms, in which heterocyclic ring each ring oxygen or sulfur atom is bonded to 2 ring carbon atoms, the optional substituents of the said heterocyclic ring being selected from the group consisting of halogen, C₁-C₆alkyl, halo-C₁-C₆alkyl, hydroxy-C₁-C₆alkyl, C(O) -C₁-C₆alkyl and oxo,
in free form or in salt form,
for use as a medicament.

2. The use of a quinazolinone compound of the formula
(I) as defined in claim 1, for the manufacture of a medicament for the treatment or prevention of a disease or condition, in which vanilloid receptor activation plays a role or is implicated.

3. A pharmaceutical composition comprising a compound as defined in claim 1 of the formula I, in free form or in pharmaceutically acceptable salt form, in association with a pharmaceutical carrier or diluent.

4. A combination comprising a therapeutically effective amount of a compound as defined in claim 1 of the formula I, in free form or in pharmaceutically acceptable salt form, and a second drug substance, for simultaneous or sequential administration.

5. A quinazolinone compound of the formula 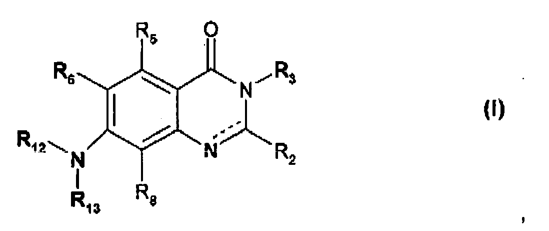 wherein
---- is a single bond or a double bond;
R₂ is selected from
(a) C₁-C₈alkyl, C₃-C₆cycloalkyl, (C₁-C₆alkyl)amino or di-(C₁-C₆alkyl)amino;
or
(b) NH₂, hydroxyC₁-C₆alkylamino-, aminoC₁-C₆alkylamino, C₂-C₆alkenyl, di(trifluoromethyl)C₁-C₆alkyl, R₉-O-(C₁ -C₆alkyl)- in which the alkyl chain is optionally substituted by trifluoromethyl, (NC)-C₁ -C₆alkyl-, (R₁₀R₁₁N-)C₁ - C₆alkyl-, (C₁ -C₆alkyl)-SO₂-(C₁ -C₆alkyl)-, wherein R₉, R₁₀ and R₁₁ are each independently H or C₁-C₆ alkyl; phenyl optionally substituted by one, two or three substituents each independently selected from the group consisting of halogen, C₁ -C₆alkyl, halogen-substituted C₁-C₆alkyl, hydroxy C₁-C₆alkyl, cyano or a group -(C=O)-R₂ₐ, where R₂ₐ is C₁ -C₆alkyl; or 5, 6, or 7-membered, saturated or unsaturated, heterocyclic ring, directly attached to the quinazolinone ring or attached through -C₁ -C₆ alkyl-, containing one, two, or three heteroatoms selected from N, O and S, and optionally substituted with one, two or three substituents selected from C₁ -C₆alkyl, C₁ -C₆alkoxy, hydroxy, cyano, halo, R₁₀R₁₁N-, R₉-O-(C=O)-, -(C=O)-N-R₁₀R₁₁, =O and phenyl ;
R₃ is selected from
(a'):
phenyl substituted by one, two or three substituents each independently selected from the group consisting of halogen, C₁-C₆alkyl, halogen-substituted C₁-C₆alkyl, hydroxyC₁-C₆alkyl, cyano or a group -C(=O)-R₃ₐ, where R₃ₐ is C₁-C₆alkyl; or
(b'):
C₁ -C₆alkyl, (NC)-C₁ -C₆alkyl-, R₉-O-(C₁ -C₆alkyl)-, R₉-O-( C₁ -C₆alkyl)-O-(C₁ - C₆alkyl)-,R₁₀R₁₁N-(C₁-C₆alkyl)-, R₁₀R₁₁N-(C=O)-(C₁-C₆alkyl)-, or (C₁ - C₆alkyl)-SO₂-(C₁-C₆alkyl)-, wherein R₉, R₁₀ and R₁₁ are each independently H or C₁-C₆ alkyl; or
unsubstituted phenyl, phenyl substituted with one or two substituents selected from -(C₁ -C₆alkoxy)-, R₁₀R₁₁N-, R₁₀R₁₁N-(C₁-C₆alkyl)-, -SO₂-(C₁-C₆alkyl), R₉-O-(C=O)-, wherein R₉, R₁₀ and R₁₁ are as defined above, or with halo-substituted phenyl or a 5- or 6-membered saturated or unsaturated heterocyclic ring having one, two or three heteroatoms selected from N, O and S and optionally including a further substituent selected from halo, or phenyl substituted with three or four substituents selected from halo, hydroxyl, and C₁ -C₆alkyl; or
a cycloalkyl ring having 3, 4, 5 or 6 carbon atoms, directly attached to the quinazolinone ring or attached through -C₁ -C₆alkyl-, and which is optionally substituted with one or two substituents selected from C₁ -C₆alkyl, C₁-C₆alkoxy, hydroxy, cyano, halo, R₁₀R₁₁N-, R₉-O-(C=O)-, -(C=O)-N-R₁₀R₁₁, and phenyl; or
benzyl, or phenyl(C₁ -C₆alkyl)-, phenoxy-( C₁ -C₆alkyl)- or phenyl(C=O)-(C₁ - C₆alkyl)-, optionally substituted with one, two, or three substituents selected from C₁-C₆alkyl, C₁ -C₆alkoxy, hydroxy, cyano, halo, R₁₀R₁₁N-, R₉-O-(C=O)-, -(C=O)-N-R₁₀R₁₁, and phenyl; or
a 5, 6, or 7- membered, saturated or unsaturated, heterocyclic ring, directly attached to the quinazolinone ring or attached through -C₁ -C₆ alkyl-, containing one, two, or three heteroatoms selected from N, O and S, and optionally substituted with one, two or three substituents selected from C₁ - C₆alkyl, C₁-C₆alkoxy, hydroxy, cyano, halo, R₁₀R₁₁N-, R₉-O-(C=O)-, -(C=O)-N-R₁₀R₁₁, =O and phenyl; or
a 9- or 10- membered aromatic or heterocyclic fused ring, directly attached to the quinazolinone ring or attached through -C₁ -C₆ alkyl-, containing zero, one, two or three heteroatoms selected from N, O and S, and optionally substituted with one, two, three or four substituents selected from C₁ -C₆alkyl, C₁ -C₆alkoxy, hydroxy, cyano, halo, R₁₀R₁₁N-, R₉-O-(C=O)-, -(C=O)-N-R₁₀R₁₁, and phenyl; and
R₅ and R₆ are each independently hydrogen, halogen, C₁-C₆alkyl, C₂-C₆alkenyl, C₂-C₆alkynyl, hydroxy, hydroxy-substituted C₁-C₆alkyl, C₁-C₆alkoxy, C₃-C₆cycloalkyl, cyano, -C(=O)H, phenyl, (C₃-C₆cycloalkyl)C₁-C₆alkyl, (C₃-C₆cycloalkyl)C₁-C₆alkoxy, (C₁-C₆alkoxycarbonylamino)C₁-C₆alkoxy or (C₁-C₆alkylcarbonylamino)C₁-C₆alkoxy, (amino) C₁-C₆alkoxy, (dimethylamino)C₁-C₆alkoxy, or (C₁-C₆alkoxycarbonyl) C₁-C₆alkoxy;
R12 is hydrogen, formyl, C₁-C₆alkylcarbonyl or benzyl, the phenyl group of which is optionally substituted by 1, 2 or 3 substituents, selected from the group consisting of halogen, C₁-C₆alkyl, halo-C₁-C₆alkyl, hydroxy, hydroxy-C₁-C₆alkyl, C₁-C₆alkoxy, C₁-C₆alkoxy-C₁-C₆alkyl, halo-C₁-C₆alkoxy, C₁-C₆alkylthio, halo-C₁-C₆alkylthio, C₁-C₆alkylsulfinyl, halo-C₁-C₆alkylsulfinyl, C₁-C₆alkylsulfonyl, halo-C₁-C₆alkylsulfonyl, C₃-C₆cycloalkyl, C₃-C₆cycloalkyl-C₁-C₆alkyl, C₃-C₆cycloalkoxy, C₃-C₆cycloalkoxy-C₁-C₆alkyl, amino, C₁-C₆alkylamino, di-(C₁-C₆alkyl)amino, C₁-C₆alkoxycarbonylamino, cyano, formyl and C₁-C₆alkylcarbonyl, or is substituted at two adjacent carbon atoms by -O-CH₂-O- or -O-CF₂-O-; and
R13 and R8, taken together, represent, together with the three-membered moiety -N-C-C-, to which they are attached, a five-, six-, seven- or eight-membered, partially or fully unsaturated, optionally substituted, heterocyclic ring, which contains 1 ring nitrogen atom and optionally either 1 further ring nitrogen, oxygen or sulfur atom or 2 further ring nitrogen atoms, in which heterocyclic ring each ring oxygen or sulfur atom is bonded to 2 ring carbon atoms, the optional substituents of the said heterocyclic ring being selected from the group consisting of halogen, C₁-C₆alkyl, halo-C₁-C₆alkyl, hydroxy-C₁-C₆alkyl, C(O) -C₁-C₆alkyl and oxo,
in free form or in salt form,
provided that the compound of formula I is not
pyrido[2,3-h]quinazolin-4(3H)-one, 2,3-diphenyl;
pyrido[2,3-h]quinazolin-4(3H)-one, 3-(4-nitrophenyl)-2-phenyl;
pyrido[2,3-h]quinazolin-4(3H)-one, 3-(4-methylphenyl)-2-phenyl;
pyrido[2,3-h]quinazolin-4(3H)-one, 3-(2-methylphenyl)-2-phenyl;
pyrido[2,3-h]quinazolin-4(3H)-one, 2-(4-chlorophenyl)-3-phenyl;
pyrido[2,3-h]quinazolin-4(3H)-one, 2-(4-chlorophenyl)-3-(4-nitrophenyl);
pyrido[2,3-h]quinazolin-4(3H)-one, 2-(4-chlorophenyl)-3-(4-methylphenyl);
pyrido[2,3-h]quinazolin-4(3H)-one, 2-(4-chlorophenyl)-3-(2-methylphenyl);
pyrido[2,3-h]quinazolin-4(3H)-one, 2-(4-methylphenyl)-3-phenyl;
pyrido[2,3-h]quinazolin-4(3H)-one, 2-(4-methylphenyl)-3-(4-nitrophenyl);
pyrido[2,3-h]quinazolin-4(3H)-one, 2,3-bis(4-methylphenyl);
pyrido[2,3-h]quinazolin-4(3H)-one, 3-(2-methylphenyl)-2-(4-methylphenyl);
pyrido[2,3-h]quinazolin-4(3H)-one, 2-(4-methoxyphenyl)-3-phenyl;
pyrido[2,3-h]quinazolin-4(3H)-one, 2-(4-methoxyphenyl)-3-(4-nitrophenyl);
pyrido[2,3-h]quinazolin-4(3H)-one, 2-(4-methoxyphenyl)-3-(4-methylphenyl);
pyrido[2,3-h]quinazolin-4(3H)-one, 2-(4-methoxyphenyl)-3-(2-methylphenyl);
pyrido[2,3-h]quinazolin-4(3H)-one, 2-(4-methylphenyl);
pyrido[2,3-h]quinazolin-4(3H),one, 2-(4-methylphenyl)-3-(phenylmethyl);
pyrido[2,3-h]quinazolin-4(3H)-one, 3-methyl-2-(4-methylphenyl);
pyrido[2,3-h]quinazolin-4(3H)-one, 3-phenyl-2-(2-phenylethenyl);
pyrido[2,3-h]quinazolin-4(3H)-one, 2-[2-(4-methoxyphenyl)ethenyl]-3-phenyl;
pyrido[2,3-h]quinazolin-4(3H)-one, 2-[2-(4-methylphenyl)ethenyl]-3-phenyl;
pyrido[2,3-h]quinazolin-4(3H)-one, 2-[2-(4-chlorophenyl)ethenyl]-3-phenyl;
pyrido[2,3-h]quinazolin-4(3H)-one, 2-[2-(4-nitrophenyl)ethenyl]-3-phenyl;
pyrido[2,3-h]quinazolin-4(3H)-one, 3-(4-methoxyphenyl)-2-(2-phenylethenyl);
pyrido[2,3-h]quinazolin-4(3H)-one, 3-(4-methylphenyl)-2-(2-phenylethenyl);
pyrido[2,3-h]quinazolin-4(3H)-one, 3-(3-nitrophenyl)-2-(2-phenylethenyl);
pyrido[2,3-h]quinazolin-4(3H)-one, 3-(4-methoxyphenyl)-2-[2-(4-methoxyphenyl)ethenyl];
pyrido[2,3-h]quinazolin-4(3H)-one, 3-(4-methoxyphenyl)-2-[2-(4-methylphenyl)ethenyl];
pyrido[2,3-h]quinazolin-4(3H)-one, 3-(4-chlorophenyl)-2-[2-(4-chiorophenyl)ethenyl];
pyrido[2,3-h]quinazolin-4(3H)-one, 2-methyl-3-phenyl;
pyrido[2,3-h]quinazolin-4(3H)-one, 3-(4-methoxyphenyl)-2-methyl;
pyrido[2,3-h]quinazolin-4(3H)-one, 2-methyl-3-(2-methylphenyl);
pyrido[2,3-h]quinazolin-4(3H)-one, 2-methyl-3-(4-methylphenyl);
pyrido[2,3-h]quinazolin-4(3H)-one, 3-(2-chlorophenyl)-2-methyl;
pyrido[2,3-h]quinazolin-4(3H)-one, 3-(4-chlorophenyl)-2-methyl; pyrido[2,3-h]quinazolin-4(3H)-one, 2-methyl-3-(phenylmethyl)

## Patentansprüche

1. Chinazolinonverbindung, der Formel 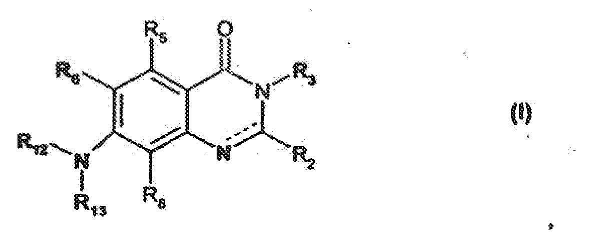 wobei
---- eine Einfachbindung oder eine Doppelbindung ist;
R₂ ausgewählt ist aus
(a) C₁-C₈-Alkyl, C₃-C₆-Cycloalkyl, (C₁-C₆-Alkyl)amino und Di(C₁-C₆-alkyl)amino;
oder
(b) NH₂, Hydroxy-C₁-C₆-alkylamino, Amino-C₁-C₆-alkylamino, C₂-C₆-Alkenyl, Di(trifluormethyl)-C₁-C₆-alkyl, R₉-O-(C₁-C₆-Alkyl)-, wobei die Alkylkette gegebenenfalls substituiert ist mit Trifluormethyl, (NC)-C₁-C₆-Alkyl-, (R₁₀R₁₁N-)-C₁-C₆-Alkyl-, (C₁-C₆-Alkyl)-SO₂-(C₁-C₆-alkyl)-, wobei R₉, R₁₀ und R₁₁ jeweils unabhängig H oder C₁-C₆-Alkyl sind; Phenyl, gegebenenfalls substituiert mit einem, zwei oder drei Substituenten, jeweils unabhängig ausgewählt aus der Gruppe, bestehend aus Halogen, C₁-C₆-Alkyl, halogensubstituiertem C₁-C₆-Alkyl, Hydroxy-C₁-C₆-alkyl, Cyano oder einem Rest -(C=O)-R₂ₐ, wobei R₂ₐ C₁-C₆-Alkyl ist; oder einem 5-, 6- oder 7-gliedrigen, gesättigten oder ungesättigten heterocyclischen Ring, der direkt an den Chinazolinonring gebunden ist oder über -C₁-C₆-Alkyl- gebunden ist, umfassend ein, zwei oder drei Heteroatome, ausgewählt aus N, O und S, gegebenenfalls substituiert mit einem, zwei oder drei Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁, =O und Phenyl;
R₃ ausgewählt ist aus
(a'):
Phenyl, substituiert mit einem, zwei oder drei Substituenten, jeweils unabhängig ausgewählt aus der Gruppe, bestehend aus Halogen, C₁-C₆-Alkyl, halogensubstitutem C₁-C₆-Alkyl, Hydroxy-C₁-C₆-alkyl, Cyano und einem Rest -C(=O)-R₃ₐ, wobei R₃ₐ C₁-C₆-Alkyl ist; oder
(b'):
C₁-C₆-Alkyl, (NC)-C₁-C₆-Alkyl-, R₉-O-(C₁-C₆-Alkyl)-, R₉-O-(C₁-C₆-Alkyl)-O-(C₁-C₆-alkyl)-, R₁₀R₁₁N-(C₁-C₆-Alkyl)-, R₁₀R₁₁N-(C=O)-(C₁-C₆-Alkyl)- und (C₁-C₆-Alkyl)-SO₂-(C₁-C₆-alkyl)-, wobei R₉, R₁₀ und R₁₁ jeweils unabhängig H oder C₁-C₆-Alkyl sind; oder
nichtsubstituiertem Phenyl, Phenyl, substituiert mit einem oder zwei Substituenten, ausgewählt aus -(C₁-C₆-Alkoxy)-, R₁₀R₁₁N-, R₁₀R₁₁N-(C₁-C₅-Alkyl)-, - SO₂-(C₁-C₆-Alkyl)-, R₉-O-(C=O)-, wobei R₉, R₁₀ und R₁₁ wie vorstehend definiert sind, oder mit halogensubstituiertem Phenyl oder einem 5- oder 6-gliedrigen, gesättigten oder ungesättigten heterocyclischen Ring mit einem, zwei oder drei Heteroatomen, ausgewählt aus N, O und S und gegebenenfalls umfassend einen weiteren Substituenten, ausgewählt aus Halogen, oder Phenyl, substituiert mit drei oder vier Substituenten, ausgewählt aus Halogen, Hydroxy und C₁-C₆-Alkyl; oder
einem Cycloalkylring mit 3, 4, 5 oder 6 Kohlenstoffatomen, direkt an dem Chinazolinonring gebunden oder über -C₁-C₆-Alkyl- gebunden, welcher gegebenenfalls mit einem oder zwei Substituenten substituiert ist, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁ und Phenyl; oder
Benzyl, oder Phenyl(C₁-C₆-alkyl)-, Phenoxy(C₁-C₆-alkyl)- oder Phenyl(C=O)-(C₁-C₆-alkyl)-, gegebenenfalls substituiert mit einem, zwei oder drei Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁ und Phenyl; oder
einem 5-, 6- oder 7-gliedrigen, gesättigten oder ungesättigten heterocyclischen Ring, direkt an den Chinazolinonring gebunden oder über -C₁-C₆-Alkyl-gebunden, umfassend ein, zwei oder drei Heteroatome, ausgewählt aus N, O und S, gegebenenfalls substituiert mit einem, zwei oder drei Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁, =O und Phenyl; oder
einem 9- oder 10-gliedrigen aromatischen oder heterocyclisch kondensierten Ring, direkt an den Chinazolinonring gebunden oder über -C₁-C₆-Alkyl-gebunden, umfassend null, ein, zwei oder drei Heteroatome, ausgewählt aus N, O und S, gegebenenfalls substituiert mit einem, zwei, drei oder vier Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁, und Phenyl; und
R₅ und R₆ jeweils unabhängig Wasserstoff, Halogen, C₁-C₆-Alkyl, C₂-C₆-Alkenyl, C₂-C₆-Alkinyl, Hydroxy, hydroxysubstituiertes C₁-C₆-Alkyl, C₁-C₆-Alkoxy, C₃-C₆-Cycloalkyl, Cyano, -C(=O)H, Phenyl, (C₃-C₆-Cycloalkyl)-C₁-C₆-alkyl, (C₃-C₆-Cycloalkyl)-C₁-C₆-alkoxy, (C₁-C₆-Alkoxycarbonylamino)-C₁-C₆-alkoxy oder (C₁-C₆-Alkylcarbonylamino)-C₁-C₆-alkoxy, (Amino)-C₁-C₆-alkoxy, (Dimethylamino)-C₁-C₆-alkoxy oder (C₁-C₆-Alkoxycarbonyl)-C₁-C₆-alkoxy sind;
R12 Wasserstoff, Formyl, C₁-C₆-Alkylcarbonyl oder Benzyl ist, dessen Phenylgruppe gegebenenfalls substituiert ist mit 1, 2 oder 3 Substituenten, ausgewählt aus der Gruppe, bestehend aus Halogen, C₁-C₆-Alkyl, Halogen-C₁-C₆-alkyl, Hydroxy, Hydroxy-C₁-C₆-alkyl, C₁-C₆-Alkoxy, C₁-C₆-Alkoxy-C₁-C₆-alkyl, Halogen-C₁-C₆-alkoxy, C₁-C₆-Alkylthio, Halogen-C₁-C₆-alkylthio, C₁-C₆-Alkylsulfinyl, Halogen-C₁-C₆-alkylsulfinyl, C₁-C₆-Alkylsulfonyl, Halogen-C₁-C₆-alkylsulfonyl, C₃-C₆-Cycloalkyl, C₃-C₆-Cycloalkyl-C₁-C₆-alkyl, C₃-C₆-Cycloalkoxy, C₃-C₆-Cycloalkoxy-C₁-C₆-alkyl, Amino, C₁-C₆-Alkylamino, Di(C₁-C₆-alkyl)amino, C₁-C₆-Alkoxycarbonylamino, Cyano, Formyl und C₁-C₆-Alkylcarbonyl, oder an zwei benachbarten Kohlenstoffatomen mit -O-CH₂-O- oder -O-CF₂-O-substituiert ist; und
R13 und R8 zusammengenommen zusammen mit der dreigliedrigen Einheit -N-C-C-, an die sie gebunden sind, einen fünf-, sechs-, sieben- oder achtgliedrigen, teilweise oder vollständig ungesättigten, gegebenenfalls substituierten heterocyclischen Ring bilden, der 1 Stickstoff-Ringatom enthält und gegebenenfalls entweder 1 weiteres Stickstoff-, Sauerstoff- oder Schwefel-Ringatom enthält oder 2 weitere Stickstoff-Ringatome enthält, wobei in dem heterocyclischen Ring jedes Sauerstoff- oder Schwefel-Ringatom an 2 Kohlenstoff-Ringatome gebunden ist, wobei die wählbaren Substituenten des heterocyclischen Rings ausgewählt sind aus der Gruppe, bestehend aus Halogen, C₁-C₆-Alkyl, Halogen-C₁-C₆-alkyl, Hydroxy-C₁-C₆-alkyl, C(O)-C₁-C₆-alkyl und Oxo,
in freier Form oder in Salzform
für die Verwendung als Medikament.

2. Verwendung einer Chinazolinonverbindung der wie in Anspruch 1 definierten Formel (I) für die Herstellung eines Medikaments für die Behandlung oder Vorbeugung einer Erkrankung oder eines Zustands, bei der/dem Vanilloidrezeptor-Aktivierung eine Rolle spielt oder beteiligt ist.

3. Pharmazeutische Zusammensetzung, umfassend eine Verbindung der in Anspruch 1 definierten Formel (I) in freier Form oder in pharmazeutisch verträglicher Salzform in Verbindung mit einem pharmazeutischen Träger oder Verdünnungsmittel.

4. Kombination, umfassend eine therapeutisch wirksame Menge einer Verbindung der in Anspruch 1 definierten Formel (I) in freier Form oder in pharmazeutisch verträglicher Salzform und einen zweiten Arzneistoff für die gleichzeitige oder aufeinander folgende Verabreichung.

5. Chinazolinonverbindung der Formel 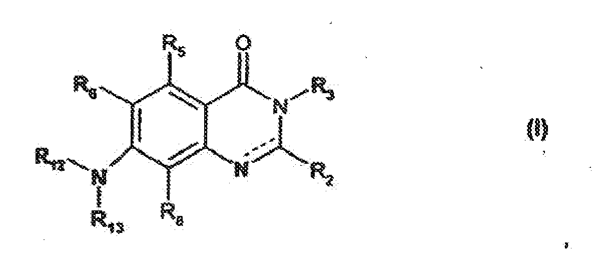 wobei
---- eine Einfachbindung oder eine Doppelbindung ist;
R₂ ausgewählt ist aus
(a) C₁-C₈-Alkyl, C₃-C₆-Cycloalkyl, (C₁-C₆-Alkyl)amino und Di(C₁-C₆-alkyl)amino;
oder
(b) NH₂, Hydroxy-C₁-C₆-alkylamino, Amino-C₁-C₆-alkylamino, C₂-C₆-Alkenyl, Di(trifluormethyl)-C₁-C₆-alkyl, R₉-O-(C₁-C₆-Alkyl)-, wobei die Alkylkette gegebenenfalls substituiert ist mit Trifluormethyl, (NC)-C₁-C₆-Alkyl-, (R₁₀R₁₁N-)-C₁-C₆-Alkyl-, (C₁-C₆-Alkyl)-SO₂-(C₁-C₆-alkyl)-, wobei R₉, R₁₀ und R₁₁ jeweils unabhängig H oder C₁-C₆-Alkyl sind; Phenyl, gegebenenfalls substituiert mit einem, zwei oder drei Substituenten, jeweils unabhängig ausgewählt aus der Gruppe, bestehend aus Halogen, C₁-C₆-Alkyl, halogensubstituiertem C₁-C₆-Alkyl, Hydroxy-C₁-C₆-alkyl, Cyano oder einem Rest -(C=O)-R₂ₐ, wobei R₂ₐ C₁-C₆-Alkyl ist; oder einem 5-, 6- oder 7-gliedrigen, gesättigten oder ungesättigten heterocyclischen Ring, der direkt an den Chinazolinonring gebunden ist oder über -C₁-C₆-Alkyl- gebunden ist, umfassend ein, zwei oder drei Heteroatom, ausgewählt aus N, 0 und S, gegebenenfalls substituiert mit einem, zwei oder drei Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁, =O und Phenyl;
R₃ ausgewählt ist aus
(a'):
Phenyl, substituiert mit einem, zwei oder drei Substituenten, jeweils unabhängig ausgewählt aus der Gruppe, bestehend aus Halogen, C₁-C₆-Alkyl, halogerisubstitutem C₁-C₆-Alkyl, Hydroxy-C₁-C₆-alkyl, Cyano und einem Rest -C(=O)-R₃ₐ, wobei R₃ₐ C₁-C₆-Alkyl ist; oder
(b'):
C₁-C₆-Alkyl, (NC)-C₁-C₆-Alkyl-, R₉-O-(C₁-C₆-Alkyl)-, R₉-O-(C₁-C₆-Alkyl)-O-(C₁-C₆-alkyl)-, R₁₀R₁₁N-(C₁-C₆-Alkyl)-, R₁₀R₁₁N-(C=O)-(C₁-C₆-Alkyl)- und (C₁-C₆-Alkyl)-SO₂-(C₁-C₆-alkyl)-, wobei R₉, R₁₀ und R₁₁ jeweils unabhängig H oder C₁-C₆-Alkyl sind; oder nichtsubstituiertem Phenyl, Phenyl, substituiert mit einem oder zwei Substituenten, ausgewählt aus -(C₁-C₆-Alkoxy)-, R₁₀R₁₁N-, R₁₀R₁₁N-(C₁-C₆-Alkyl)-, SO₂-(C₁-C₆-Alkyl), R₉-O-(C=O)-, wobei R₉, R₁₀ und R₁₁ wie vorstehend definiert sind, oder mit halogensubstituiertem Phenyl oder einem 5- oder 6-gliedrigen, gesättigten oder ungesättigen heterocyclischen Ring mit einem, zwei oder drei Heteroatomen, ausgewählt aus N, O und S und gegebenenfalls umfassend einen weiteren Substituenten, ausgewählt aus Halogen, oder Phenyl, substituiert mit drei oder vier Substituenten, ausgewählt aus Halogen, Hydroxy und C₁-C₆-Alkyl; oder
einem Cycloalkylring mit 3, 4, 5 oder 6 Kohlenstoffatomen, direkt an dem Chinazolinonring gebunden oder über -C₁-C₆-Alkyl- gebunden, welcher gegebenenfalls mit einem oder zwei Substituenten substituiert ist, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁ und Phenyl; oder
Benzyl, oder Phenyl(C₁-C₆-alkyl)-, Phenoxy(C₁-C₆-alkyl)- oder Phenyl(C=O)-(C₁-C₆-alkyl)-, gegebenenfalls substituiert mit einem, zwei oder drei Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁ und Phenyl; oder
einem 5-, 6- oder 7-gliedrigen, gesättigten oder ungesättigten heterocyclischen Ring, direkt an den Chinazolinonring gebunden oder über -C₁-C₆-Alkyl-gebunden, umfassend ein, zwei oder drei Heteroatome, ausgewählt aus N, O und S, gegebenenfalls substituiert mit einem, zwei oder drei Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁, =O und Phenyl; oder
einem 9- oder 10-gliedrigen aromatischen oder heterocyclisch kondensierten Ring, direkt an den Chinazolinonring gebunden oder über -C₁-C₆-Alkyl-gebunden, umfassend mull, ein, zwei oder drei Heteroatome, ausgewählt aus N, O und S, gegebenenfalls substituiert mit einem, zwei, drei oder vier Substituenten, ausgewählt aus C₁-C₆-Alkyl, C₁-C₆-Alkoxy, Hydroxy, Cyano, Halogen, R₁₀R₁₁N-, R₉-O-(C=O)-, -(C=O)-N-R₁₀R₁₁, und Phenyl; und
R₅ und R₆ jeweils unabhängig Wasserstoff, Halogen, C₁-C₆-Alkyl, C₂-C₆-Alkenyl, C₂-C₆-Alkinyl, Hydroxy, hydroxysubstituiertes C₁-C₆-Alkyl, C₁-C₆-Alkoxy, C₃-C₆-Cycloalkyl, Cyano, -C(=O)H, Phenyl, (C₃-C₆-Cycloalkyl)-C₁-C₆-alkyl, (C₃-C₆-Cycloalkyl)-C₁-C₆-alkoxy, (C₁-C₆-Alkoxycarbonylamino)-C₁-C₆-alkoxy oder (C₁-C₆-Alkylcarbonylamino)-C₁-C₆-alkoxy, (Amino)-C₁-C₆-alkoxy, (Dimethylamino)-C₁-C₆-alkoxy oder (C₁-C₆-Alkoxycarhonyl)-C₁-C₆-alkoxy sind;
R12 Wasserstoff, Formyl, C₁-C₆-Alkylcarbonyl oder Benzyl ist, dessen Phenylgruppe gegebenenfalls substituiert ist mit 1, 2 oder 3 Substituenten, ausgewählt aus der Gruppe, bestehend aus Halogen, C₁-C₆-Alkyl, Halogen-C₁-C₆-alkyl, Hydroxy, Hydroxy-C₁-C₆-alkyl, C₁-C₆-Alkoxy, C₁-C₆-Alkoxy-C₁-C₆-alkyl Halogen-C₁-C₆-alkoxy, C₁-C₆-Alkylthio, Halogen-C₁-C₆-alkylthio, C₁-C₆-Alkylsulfinyl, Halogen-C₁-C₆-alkylsulfinyl, C₁-C₆-Alkylsulfonyl, Halogem-C₁-C₆-alkylsulfonyl, C₃-C₆-Cycloalkyl, C₃-C₆-Cycloalkyl-C₁-C₆-alkyl, C₃-C₆-Cycloalkoxy, C₃-C₆-Cycloalkoxy-C₁-C₆-alkyl, Amino, C₁-C₆-Alkylamino, Di(C₁-C₆-alkyl)amino, C₁-C₆-Alkoxycarbonylamino, Cyano, Formyl und C₁-C₆-Alkylcarbonyl, oder an zwei benachbarten Kohlenstoffatomen mit -O-CH₂-O- oder -O-CF₂-O-substituiert ist; und
R13 und R8 zusammengenommen zusammen mit der dreigliedrigen Einheit -N-C-C-, an die sie gebunden sind, einen fünf-, sechs-, sieben- oder achtgliedrigen, teilweise oder vollständig ungesättigten, gegebenenfalls substituierten heterocyclischen Ring bilden, der 1 Stickstoff-Ringatom enthält und gegebenenfalls entweder 1 weiteres Stickstoff-, Sauerstoff- oder Schwefel-Ringatom enthält oder 2 weitere Stickstoff-Ringatome enthält, wobei in dem heterocyclischen Ring jedes Sauerstoff- oder Schwefel-Ringatom an 2 Kohlenstoff-Ringatome gebunden ist, wobei die wählbaren Substituenten des heterocyclischen Rings ausgewählt sind aus der Gruppe, bestehend aus Halogen, C₁-C₆)-Alkyl, Halogen-C₁-C₆-alkyl, Hydroxy-C₁-C₆-alkyl, C(O)-C₁-C₆-alkyl und Oxo,
in freier Form oder in Salzform,
vorausgesetzt, dass die Verbindung der Formel I nicht Pyrido[2,3-h]chinazolin-4(3H)-on, 2,3-biphenyl;
Pyrido[2,3-h]chinazolin-4(3H)-on, 3-(4-nitrophenyl)-2-phenyl;
Pyrido[2,3-h]chinazolin-4(3H)-on, 3-(4-methylphenyl)-2-phenyl;
Pyrido[2,3-h]chinasolin-4(3H)-on, 3-(2-methylphenyl)-2-phenyl;
Pyrido[3,3-h]chinazolin-4(3H)-on, 2-(4-chlorphenyl)-3-phenyl;
Pyrido[2,3-n]chinazolin-4(3H)-on, 2-(4-chlorphenyl)-3-(4-nitrophenyl);
Pyrido[2,3-h]chinazolin-4(3H)-on, 2-(4-chlorphenyl)-3-(4-methylphenyl);
Pyrido[2,3-h]chinazolin-4(3H)-on, 2-(4-chlorphenyl)-3-(2-methylphenyl);
Pyrido[2,3-h]chinazolin-4(3H)-on, 2-(4-methylphenyl)-3-phenyl;
Pyrido[2,3-h]chinazolin-4(3H)-on, 2-(4-methylphenyl)-3-(4-nitrophenyl);
Pyrido[2,3-h]chinazolin-4(3H)-on, 2,3-bis(4-methylphenyl);
Pyrido[2,3-h]chinazolin-4(3H)-on, 3-(2-methylphenyl) -2-(4-methylphenyl);
Pyrido[2,3-h]chinazolin-4(3H)-on, 2-(4-methoxyphenyl)-3-phenyl;
Pyrido[2,3-h]chinazolin-4(3H)-on, 2-(4-methoxyphenyl)-3-(4-nitrophenyl); Pyrido[2,3-h]chinazolin-4(3H)-on, 2-(4-methoxyphenyl)-3-(4-methylphenyl); Pyrido[2,3-h]chinazolin-4(3H)-on, 2-(4-methoxyphenyl)-3-(2-methylphenyl); Pyrido[2,3-h]chinazolin-4(3H)-on, 2-(4-methylphenyl); Pyrido[2,3-h]chinazolin-4(3H)-on, 2-(4-methylphenyl)-3-(phenylmethyl);
Pyrido[2,3-h]chinazolin-4(3H)-on, 3-methyl-2-(4-methylphenyl);
Pyrido[2,3-h]chinazolin-4(3H)-on, 3-phenyl-2-(2-phenylethenyl);
Pyrido[2,3-h]chinazolin-4(3H)-on, 2-[3-(4-methoxyphenyl)ethenyl]-3-phenyl; Pyrido[2,3-h]chinazolin-4(3H)-on, 2-[2-(4-methylphenyl)ethenyl]-3-pheryl; Pyrido[2,3-h]chinazolin-4(3H)-on, 2-[2-(4-chlorphenyl)ethenyl]-3-phenyl; Pyrido[2,3-h]chinazolin-4(3H)-on, 2-[2-(4-nitrophenyl)ethenyl]-3-phenyl; Pyrido[2,3-h]chinazolin-4(3H)-on, 3-(4-methoxyphenyl)-2-(2-phenylethenyl); Pyrido[2,3-h]chinazolin-4(3H)-on, 3-(4-methylphenyl)-2-(2-phenylethenyl); Pyrido[2,3-h]chinazolin-4(3H)-on, 3-(3-nitrophenyl)-2-(2-phenylethenyl); Pyrido[2,3-h]chinazolin-4(3H)-on, 3-(4-methoxyphenyl)-2-[2-(4-methoxyphenyl)ethenyl]; Pyrido[2,3-h]chinazolin-4(3H)-on, 3-(4-methoxyphenyl)-2-[2-(4-methylphenyl)ethenyl]; Pyrido[2,3-h]chinazolin-4(3H)-on, 3-(4-chlorphenyl)-2-[2-(4-chlorphenyl)ethenyl];
Pyrido[2,3-h]chinazolin-4(3H)-on, 2-methyl-3-phenyl;
Pyrido[2,3-h]chinazolin-4(3H)-on, 3-(4-methoxyphenyl)-2-methyl;
Pyrido[2,3-h]chinazolin-4(3H)-on, 2-methyl-3-(2-methylphenyl);
Pyrido[2,3-h]chinazolin-4(3H)-on, 2-methyl-3-(4-methylphenyl);
Pyrido[2,3-h]chinazolin-4(3H)-on, 3-(2-chlorphenyl)-2-methyl;
Pyrido[2,3-h]chinazolin-4(3H)-on, 3-(4-chlorphenyl)-2-methyl;
Pyrido[2,3-h]chinazolin-4(3H)-on, 2-methyl-3-(phenylmethyl)
ist.

## Revendications

1. Quinazolone de formule 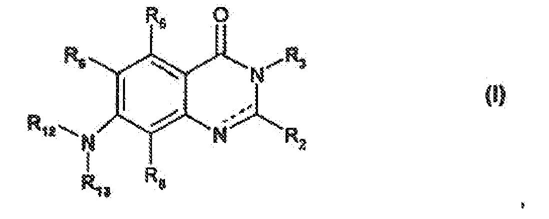 où
---- représente une liaison simple ou une liaison double ;
R₂ est choisi parmi
(a) les groupements alkyle en C₁-C₈, cycloalkyle en C₃-C₆, (alkyle en C₁-C₆)-amino ou di-(alkyle en C₁-C₆)amino ; ou
(b) les groupements NH₂, hydroxy(alkyle en C₁-C₆)-amino, amino(alkyle en C₁-C₆)-amino, alcényle en C₂-C₆, di(trifluorométhyl)-alkyle en C₁-C₆, R₉-O-(alkyle en C₁-C₆)- où la chaîne alkyle est éventuellement substituée par des groupements trifluorométhyle, (NC)-(alkyle en C₁-C₆)-, (R₁₀R₁₁N-)-(alkyle en C₁-C₆)-, (alkyle en C₁-C₆)-SO₂-(alkyle en C₁-C₆)-, où R₉, R₁₀ et R₁₁ représentent chacun de façon indépendante H ou un groupement alkyle en C₁-C₆ ; phényle éventuellement substitué par un, deux ou trois substituant indépendamment choisis dans le groupe constitué par les atomes d'halogène et les groupements alkyle en C₁-C₆, alkyle en C₁-C₆ halogéné, hydroxyalkyle en C₁-C₆, cyano ou un groupement -(C=O)-R₂ₐ, où R₂ₐ représente un groupement alkyle en C₁-C₆ ; ou un cycle hétérocyclique saturé ou non et comportant 5, 6 ou 7 chaînons, directement lié au cycle quinazolinone ou lié via un groupement -(alkyle en C₁-C₆)-, comportant un, deux ou trois hétéroatomes choisis parmi N, O et S, et éventuellement substitué par un, deux ou trois substituant choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁, =O et phényle ;
R₃ est choisi parmi
(a') :
les groupements phényle substitués par un, deux ou trois substituants indépendamment choisis dans le groupe constitué par les atomes d'halogène et les groupements alkyle en C₁-C₆, alkyle en C₁-C₆ halogéné, hydroxyalkyle en C₁-C₆, cyano ou un groupement -C(=O)-R₃ₐ, où R₃ₐ représente un groupement alkyle en C₁-C₆ ; ou
(b') :
les groupements alkyle en C₁-C₆, (NC)-(alkyle en C₁-C₆)-, R₉-O-(alkyle en C₁-C₆)-, R₉-O-(alkyle en C₁-O₆)-O-(alkyle en C₁-C₆)-, R₁₀R₁₁N-(alkyle en C₁-C₆)-, R₁₀R₁₁N-(C=O)-(alkyle en C₁-C₆)- ou (alkyle en C₁-C₆)-SO₂-(alkyle en C₁-C₆)-, où R₉, R₁₀ et R₁₁ représentent chacun de façon indépendante H ou un groupement alkyle en C₁-C₆ ; ou
les groupements phényle non substitués, phényle substitué par un ou deux substituants choisis parmi les groupements -(alkoxy en C₁-C₆)-, R₁₀R₁₁N-, R₁₀R₁₁N-(alkyle en C₁-C₆)-, -SO₂-alkyle en C₁-C₆, R₉-O-(C=O)-, où R₉, R₁₀ et R₁₁ sont tels que définis ci-avant, ou par un groupement phényle halogéné ou un cycle hétérocyclique saturé ou non et comportant 5 ou 6 chaînons ainsi qu'un, deux ou trois hétéroatomes choisis parmi N, O et S et incluant éventuellement un substituant supplémentaire choisi parmi les groupements halogéno, ou phényle substitué par trois ou quatre substituants choisis parmi les groupements halogéno, hydroxyle et alkyle en C₁-C₆ ; ou
un cycle cycloalkyle comportant 3, 4, 5 ou 6 atomes de carbone, directement lié au cycle quinazolinone ou lié via un groupement -(alkyle en C₁-C₆)-, et éventuellement substitué par un ou deux substituants choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀-R₁₁ et phényle ; ou
les groupements benzyle, ou phényl-(alkyle en C₁-C₆)-, phénoxy-(alkyle en C₁-C₆)- ou phényl(C=O)-(alkyle en C₁-C₆)-, éventuellement substitués par un, deux ou trois substituants choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁ et phényle ; ou
un cycle hétérocyclique saturé ou non et comportant 5, 6 ou 7 chaînons, directement lié au cycle quinazolinone ou lié via un groupement -(alkyle en C₁-C₆)-, comportant un, deux ou trois hétéroatomes choisis parmi N, O et S, et éventuellement substitué par un, deux ou trois substituants choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁, =O et phényle ; ou
un cycle fusionné aromatique ou hétérocyclique à 9 ou 10 chaînons, directement lié au cycle quinazolinone ou lié via un groupement -(alkyle en C₁-C₆)-, comportant zéro, un, deux ou trois hétéroatomes choisis parmi N, O et S, et éventuellement substitué par un, deux ou trois substituants choisis parmi les groupements alkyle en C₁-C₆, alkoxy en C₁-C₆, hydroxy, cyano, halogéno, R₁₀R₁₁N-, R₉-O-(C=O)-, -(C-O)-N-R₁₀R₁₁ et phényle ; et
R₅ et R₆ représentent chacun indépendamment un atome d'hydrogène ou d'halogène ou un groupement alkyle en C₁-C₆, alcényle en C₂-C₆, alcynyle en C₂-C₆, hydroxy, alkyle en C₁-C₆ substitué par hydroxy, alkoxy en C₁-C₆, cycloalkyle en C₁-C₆, cyano, -C(=O)H, phényle, (cycloalkyle en C₃-C₆)-(alkyle en C₁-C₆), (cycloalkyle en C₃-C₆)-(alkoxy en C₁-C₆), (alkoxy en C₁-C₆)-carbonylamino-(alkoxy en C₁-C₆) ou (alkyle en C₁-C₆)-carbonylamino-(alkoxy en C₁-C₆), (amino)-alkoxy en C₁-C₆, (diméthylamino)-alkoxy en C₁-C₆ ou (alkoxy en C₁-C₆)-carbonyl-(alkoxy en C₁-C₆) ;
R₁₂ représente un atome d'hydrogène ou un groupement formyle, (alkyle en C₁-C₆)-carbonyle ou benzyle, dont le groupement phényle est éventuellement substitué par 1, 2 ou 3 substituants, choisis dans le groupe constitué par les atomes d'halogène et les groupements alkyle en C₁-C₆, halogénoalkyle en C₁-C₆, hydroxy, hydroxyalkyle en C₁-C₆, alkoxy en C₁-C₆, (alkoxy en C₁-C₆)-(alkyle en C₁-C₆), halogénoalkoxy en C₁-C₆, (alkyle en C₁-C₆)-thio, halogéno-(alkyle en C₁-C₆)-thio, (alkyle en C₁-C₆)-sulfinyle, halogéno-(alkyle en C₁-C₆)-sulfinyle, (alkyle en C₁-C₆)-sulfonyle, halogéno-(alkyle en C₁-C₆)-sulfonyle, cycloalkyle en C₃-C₆, (cycloalkyle en C₃-C₆)-(alkyle en C₁-C₆), cycloalkoxy en C₃-C₆, (cycloalkoxy en C₃-C₆)-(alkyle en C₁-C₆), amino, (alkyle en C₁-C₆)-amino, di-(alkyle en C₁-C₆)-amino, (alkoxy en C₁-C₆)-carbonylamino, cyano, formyle et (alkyle en C₁-C₆)-carbonyle, ou est substitué au niveau de deux atomes de carbone adjacent par un groupement -O-CH₂-O- ou -O-CF₂-O- ; et
R₁₃ et R₈, ensemble et avec la fonction à trois atomes - N-C-C- à laquelle ils sont liés, représentent un cycle hétérocyclique éventuellement substitué, partiellement ou entièrement insaturé, à cinq, six, sept ou huit chaînons, qui comporte 1 atome d'azote de cycle et éventuellement soit 1 atome d'azote, d'oxygène ou de soufre de cycle supplémentaire, soit 2 atomes d'azote de cycle supplémentaires, chacun des atomes d'oxygène ou de soufre de cycle dudit cycle hétérocyclique étant lié à 2 atomes de carbone de cycle, les substituants éventuels dudit cycle hétérocyclique étant choisis dans le groupe constitué par les atomes d'halogène et les groupements alkyle en C₁-C₆, halogénoalkyle en C₁-C₆, hydroxyalkyle en C₁-C₆, C(O)-alkyle en C₁-C₆ et oxo, sous forme libre ou sous forme saline,
pour emploi en tant que médicament.

2. Emploi d'une quinazolinone de formule (I) conforme à la revendication 1, dans la fabrication d'un médicament destiné au traitement prophylactique ou thérapeutique d'une pathologie ou d'un état pathologique où l'activation du récepteur vanilloïde joue un rôle ou est impliquée.

3. Composition pharmaceutique comprenant un composé conforme à la revendication 1 de formule I, sous forme libre ou sous une forme saline de qualité pharmaceutique, en association avec un vecteur ou diluant de qualité pharmaceutique.

4. Combinaison comprenant une quantité thérapeutiquement active d'un composé conforme à la revendication 1 de formule I, sous forme libre ou sous une forme saline de qualité pharmaceutique, et une seconde substance médicamenteuse, pour administration simultanée ou séquentielle.

5. Quinazolinone de formule 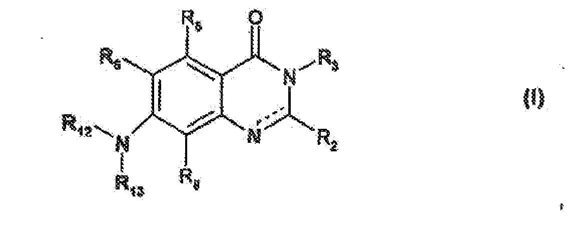 où
---- représente une liaison simple ou une liaison double ;
R₂ est choisi parmi
(a) les groupements alkyle en C₁-C₈, cycloalkyle en C₃-C₈, (alkyle en C₁-C₆)-amino ou di-(alkyle en C₁-C₆)amino ; ou
(b) les groupements NH₂, hydroxy(alkyle en C₁-C₆)-aminoamino(alkyle en C₁-C₆)-amino, alcényle en C₂-C₆, di(trifluorométhyl)-alkyle en C₁-C₆, R₉-O-(alkyle en C₁-C₆)- où la chaîne alkyle est éventuellement substituée par des groupements trifluorométhyle, (NC)-(alkyle en C₁-C₆)-, (R₁₀R₁₁N-)-(alkyle en C₁-C₆)-, (alkyle en C₁-C₆)-SO₂-(alkyle en C₁-C₆)-, où R₉, R₁₀ et R₁₁ représentent chacun de façon indépendante H ou un groupement alkyle en C₁-C₆ ; phényle éventuellement substitué par un, deux ou trois substituant indépendamment choisis dans le groupe constitué par les atomes d'halogène et les groupements alkyle en C₁-C₆, alkyle en C₁-C₆ halogéné, hydroxyalkyle en C₁-C₆, cyano ou un groupement -(C=O)-R₂ₐ, où R₂ₐ représente un groupement alkyle en C₁-C₆ ; ou un cycle hétérocyclique saturé ou non et comportant 5, 6 ou 7 chaînons, directement lié au cycle quinazolinone ou lié via un groupement -(alkyle en C₁-C₆)-, comportant un, deux ou trois hétéroatomes choisis parmi N, 0 et S, et éventuellement substitué par un, deux ou trois substituants choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁, =O et phényle ;
R₃ est choisi parmi
(a') :
les groupements phényle substitués par un, deux ou trois substituants indépendamment choisis dans le groupe constitué par les atomes d'halogène et les groupements alkyle en C₁-C₆, alkyle en C₁-C₆ halogéné, hydroxyalkyle en C₁-C₆, cyano ou un groupement -C(=O)-R₃ₐ, où R₃ₐ représente un groupement alkyle en C₁-C₆ ; ou
(b') :
les groupements alkyle en C₁-C₆, (NC)-(alkyle en C₁-C₆)-, R₉-O-(alkyle en C₁-C₆)-, R₉-O-(alkyle en C₁-C₆)-O-(alkyl en C₁-C₆)-, R₁₀R₁₁N-(alkyle en C₁-C₆)-, R₁₀R₁₁N-(C=O)-(alkyle en C₁-C₆)- ou (alkyle en C₁-C₆)-SO₂-(alkyle en C₁-C₆)-, où R₉, R₁₀ et R₁₁ représentent chacun de façon indépendante H ou un groupement alkyle en C₁-C₆ ; ou les groupements phényle non substitué, phényle substitué par un ou deux substituant choisis parmi les groupements -(alkoxy en C₁-C₆)-, R₁₀R₁₁N-, R₁₀R₁₁N-(alkyle en C₁-C₆)-, -SO₂-alkyle en C₁-C₆, R₉-O-(C=O)-, où R₉, R₁₀ et R₁₁ sont tels que définis ci-avant, ou par un groupement phényle halogéné ou un cycle hétérocyclique saturé ou non et comportant 5 ou 6 chaînons ainsi qu'un, deux ou trois hétéroatomes choisis parmi N, O et S et incluant éventuellement un substituant supplémentaire choisi parmi les groupements halogéno, ou phényle substitué par trois ou quatre substituants choisis parmi les groupements halogéno, hydroxyle et alkyle en C₁-C₆ ; ou
un cycle cycloalkyle comportant 3, 4, 5 ou 6 atomes de carbone, directement lié au cycle quinazolinone ou lié via un groupement -(alkyle en C₁-C₆)-, et éventuellement substitué par un ou deux substituants choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁ et phényle ; ou
les groupements benzoyle, ou phényl-(alkyle en C₁-C₆)-, phénoxy-(alkyle en C₁-C₆)- ou phényl (C=O)-(alkyle en C₁-C₆)-, éventuellement substitués par un, deux ou trois substituant choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁ et phényle ; ou
un cycle hétérocyclique saturé ou non et comportant 5, 6 ou 7 chaînoris, directement lié au cycle quinazolinone ou lié via un groupement -(alkyl en C₁-C₆)-, comportant un, deux ou trois hétéroatomes choisis parmi N, O et S, et éventuellement substitué par un, deux ou trois substituants choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁, =O et phényle ; ou
un cycle fusionné aromatique ou hétérocyclique à 9 ou 10 chaînons, directement lié au cycle quinazolinone ou lié via un groupement -(alkyle en C₁-C₆-, comportant zéro, un, deux ou trois hétéroatomes choisis parmi N, O et S, et éventuellement substitué par un, deux ou trois substituents choisis parmi les groupements alkyle en C₁-C₆, alkoxy en C₁-C₆, hydroxy, cyano, halogéno, R₁₀R₁₁N-, R₉-O-(C=O)-, -(C=O)-N-R₁₀R₁₁ et phényle ; et
R₅ et R₆ représentent chacun indépendamment un atome d'hydrogène ou d'halogène ou un groupement alkyle en C₁-C₆, alcényle en C₂-C₆, alcynyle en C₂-C₆, hydroxy, alkyle en C₁-C₆ substitué par hydroxy, alkoxy en C₁-C₆, cycloalkyle en C₃-C₆, cyano, -C(=O)H, phényle, (cycloalkyle en C₃-C₆)-(alkyle en C₁-C₆), (cycloalkyle en C₃-C₆) - (alkoxy en C₁-C₆), (alkoxy en C₁-C₆)-carbonylamino-(alkoxy en C₁-C₆) ou (alkyle en C₁-C₆)-carbonylamino-(alkoxy en C₁-C₆), (amino)-alkoxy en C₁-C₆, (diméthylamino)-alkoxy en C₁-C₆ ou (alkoxy en C₁-C₆)-carbonyl-(alkoxy en C₁-C₆) ;
R₁₂ représente un atome d'hydrogène ou un groupement formyle, (alkyle en C₁-C₆)-carbonyle ou benzoyle, dont le groupement phényle est éventuellement substitué par 1, 2 ou 3 substituant, choisis dans le groupe constitué par les atomes d'halogène et les groupements alkyle en C₁-C₆, halogénoalkyle en C₁-C₅, hydroxy, hydroxyalkyle en C₁-C₆, alkoxy en C₁-C₆, (alkoxy en C₁-C₆)-(alkyle en C₁-C₆), halogénoalkoxy en C₁-C₆, (alkyle en C₁-C₆)-thio, halogéno-(alkyle en C₁-C₆)-thio, (alkyle en C₁-C₆)-sulfinyle, halogéno-(alkyle en C₁-C₆)-sulfinyle, (alkyle en C₁-C₆)-sulfonyle, halogéno-(alkyle en C₁-C₆)-sulfonyle, cycloalkyle en C₃-C₆, (cycloalkyle en C₃-C₆)-(alkyle en C₁-C₆), cycloalkoxy en C₃-C₆, (cycloalkoxy en C₃-C₆)-(alkyle en C₁-C₆), amine, (alkyle en C₁-C₆)-amino, di-(alkyle en C₁-C₆)-amino, (alkoxy en C₁-C₆)-carbonylamino, cyano, formyle et (alkyle en C₁-C₆)-carbonyle, ou est substitué au niveau de deux atomes de carbone adjacent par un groupement -O-CH₂-O- ou -O-CF₂-O- ; et
R₁₃ et R₈, ensemble et avec la fonction à trois atomes - N-C-C- à laquelle ils sont liés, représentent un cycle hétérocyclique éventuellement substitué, partiellement ou entièrement insaturé, à cinq, six, sept ou huit chaînons, qui comporte 1 atome d'azote de cycle et éventuellement soit 1 atome d'azote, d'oxygène ou de soufre de cycle supplémentaire, soit 2 atomes d'azote de cycle supplémentaires, chacun des atomes d'oxygène ou de soufre de cycle dudit cycle hétérocyclique étant lié à 2 atomes de carbone de cycle, les substituants éventuels dudit cycle hétérocyclique étant choisis dans le groupe constitué par les atomes d'halogène et les groupements alkyle en C₁-C₆, halogénoalkyle en C₁-C₆, hydroxyalkyle en C₁-C₆, C(O)-alkyle en C₁-C₆ et oxo, sous forme libre ou sous forme saline,
à la condition que le composé de formule I ne soit pas l'un des composés suivants :
pyrido[2,3-h]quinazolin-4(3H)-one, 2,3-diphényl ;
pyrido[2,3-h]quinazolin-4(3H)-one, 3-(4-nitrophényl)-2-phényl ;
pyrido[2,3-h]quinazolin-4(3H)-one, 3-(4-méthylphényl)-2-phényl ;
pyrido[2,3-h]quinazolin-4(3H)-one, 3-(2-méthylphényl)-2-phényl ;
pyrido[2,3-h]quinazolin-4(3H)-one, 2-(4-chlorophényl)-3-phényl ;
pyrido[2,3-h]quinazolin-4(3H)-one, 2-(4-chlorophényl)-3-(4-nitrophényl) ;
pyrido[2,3-h]quinazolin-4(3H)-one, 2-(4-chlorophényl)-3-(4-méthylphé-nyl) ;
pyrido[2,3-h]quinazolin-4(3H)-one, 2-(4-chlorophényl)-3-(2-méthylphényl) ;
pyrido[2,3-h]quinazolin-4(3H)-one, 2-(4-méthylphényl)-3-phényl ;
pyrido[2,3-h]quinazolin-4(3H)-one, 2-(4-méthylphényl)-3-(4-nitrophényl) ;
pyrido[2,3-h]quinazolin-4(3H)-one, 2,3-bis(4-méthylphényl) ;
pyrido[2,3-h]quinazolin-4(3H)-one, 3 - (2-méthylphényl)-2-(4-métthylplényl) ;
pyrido[2,3-h]quinazolin-4(3H)-one, 2-(4-méthoxyphényl)-3-phényl ;
pyrido[2,3-h]quinasolin-4(3H)-one, 2-(4-méthoxyphényl)-3-(4-nitrophényl) ;
pyrido[2,3-h]quinazolin-4(3H)-one, 2-(4-méthoxyphényl)-3-(4-méthylphényl) ;
pyrido[2,3-h]quinazolin-4(3H)-one, 2-(4-méthoxyphényl)-3-(2-méthylphényl) ;
pyrido[2,3-h]quinazolin-4(3H)-one, 2-(4-méthylphényl) ;
pyrido[2,3-h]quinazolin-4(3H)-one, 2-(4-méthylphényl)-3-(phénylméthyl) ;
pyrid[2,3-h]quinazolin-4(3H)-one, 3-méthyl-2-(4-méthylphényl) ;
pyrido[2,3-h]quinazolin-4(3H)-one, 3-phényl-2-(2-phényléthényl) ;
pyrido[2,3-h]quinazolin-4(3H)-one, 2-[2-(4-méthoxyphényl)éthényl]-3-phényl ;
pyrido[2,3-h]quinazolin-4(3H)-one, 2-[2-(4-méthylphenyl)éthényl]-3-phényl ;
pyrido[2,3-h]quinazolin-4(3H)-one, 2-[2-(4-chlorophényl)éthényl]-3-phényl ;
pyrido[2,3-h]quinazolin-4(3H)-one, 2-[2-(4-nitrophényl)ethényl]-3-phényl ;
pyrido[2,3-h]quinazolin-4(3H)-one, 3-(4-méthoxyphényl)-2-(2-phényléthényl) ;
pyrido[2,3-h]quinazolin-4(3H)-one, 3-(4-méthylphényl)-2-(2-phényléthényl) ;
pyrido[2,3-h]quinazolin-4(3H)-one, 3-(3-nitrophényl)-2-(2-phényléthényl) ;
pyrido[2,3-h]quinazolin-4(3H)-one, 3-(4-méthoxyphényl)-2-[2-(4-méthoxyphényl)éthényl] ;
pyrido[2,3-h]quinazolin-4(3H)-one, 3-(4-méthoxyphényl)-2-(4-méthylphényl)éthényl] ;
pyrido[2,3-h]quinazolin-4-(3H)-one, 3-(4-chlorophényl)-2-[2-(4-chlorophényl)éthényl] ;
pyrido[2,3-h]quinazolin-4(3H)-one, 2-méthyl-3-phényl ;
pyrido[2,3-h]quinazolin-4(3H)-one, 3-(4-méthoxyphényl)-2-méthyl ;
pyrido[2,3-h]quinazolin-4(3H)-one, 2-méthyl-3-(2-méthylphényl) ;
pyrido[2,3-h]quinazolin-4(3H)-one, 2-méthyl-3-(4-méthylphényl) ;
pyrido[2,3-h]quinazolin-4(3H)-one, 3-(2-chlorophényl)-2-méthyl ;
pyrido[2,3-h]quinazolin-4(3H)-one, 3-(4-chlorophényl)-2-méthyl ;
pyrido[2,3-h]quinazolin-4(3H)-one, 2-méthyl-3-(phénylméthyl).
